# EUROPEAN PATENT APPLICATION

(11) **EP 4 224 163 A1**
(43) Date of publication of application: **09.08.2023**
(21) Application number: 22154836.5
(22) Date of filing: 02.02.2022
(51) Int. Cl.: G01N 33/68

(54) **DIAGNOSIS OF SEPSIS AND SYSTEMIC INFLAMMATORY RESPONSE SYNDROME BY QUANTITATIVE LC/MS METHOD**

(71) Applicant: Universitätsklinikum Jena, 07747 Jena (DE)
(72) Inventor: Kiehntopf, Michael, 07749 Jena (DE); Bigalke, Arite, 07743 Jena (DE)
(74) Representative: CH Kilger Anwaltspartnerschaft mbB

(57) **Abstract**

The invention relates to a method for quantitatively measuring a concentration of a peptide panel in a biological matrix by mass spectrometry detection, wherein multiplex multiple reaction monitoring (MRM) scanning is applied.

## Description

### Field of the invention

The present invention is in the field of medicine, more in particular diagnostics and even more in particular the diagnosis and prognosis of sepsis.

### Background

The present invention relates to the diagnosis of sepsis and septic complications. The term "sepsis" has been used to describe a variety of clinical conditions related to systemic manifestations of inflammation accompanied by an infection. Because of clinical similarities to inflammatory responses secondary to non-infectious aetiologies, identifying sepsis has been a particularly challenging diagnostic problem. According to the current definition of sepsis (Sepsis-3) as published by Singer et al. in JAMA, 2016 (doi: 10.1001/jama.2016.0287), sepsis is understood as life-threatening organ dysfunction (organ failure) caused by a dysregulated host response to infection, i.e., the sepsis results from an infectious-induced systemic inflammation, when organ failure occurs due to the host response to the underlying infection. This is in clear contrast to systemic inflammation and organ dysfunction which are not caused by an underlying infection, which is not understood as sepsis. A particular severe form of sepsis is known as septic shock, which is diagnosed by an increased lactate concentration of above 2 mmol/l in combination hypotension in absence of hypovolemia.

Despite the availability of antibiotics and supportive therapy, sepsis represents a significant cause of morbidity and mortality. Several laboratory tests have been investigated for use, in conjunction with a complete clinical examination of a subject, for the diagnosis/prognosis of sepsis (Giamarellos-Bourboulis et al., Intensive Care Med. 28: 1351-56, 2002).

Several molecular markers have been discussed to facilitate diagnosis and treatment monitoring of sepsis in humans and several animal species. The most widely used ones may be CRP (C-reactive protein) and PCT (procalcitonin) other also applied markers are pro-ADM (pro-adrenomedullin), transthyretin (TTR) and IL-6 (interleukin) among many others.

Also, various interleukins have been discussed as potential biomarkers of sepsis. However, they are of limited use at present because of a lack of specificity. For example, Carrigan et al. (Clinical Chemistry 50 (8) (2004) 1301-1314) reported the following sensitivities and specificities for these markers in humans:

| **Table 1. ROC analysis results for various biomarker-based prediction of sepsis in adult and neonatal cases.^{a} ROC analysis** | | | | | |
|---|---|---|---|---|---|
| **Marker** | **Age group** | **Cutoff range** | **Sensitivity, %** | **Specificity, %** | **Referenced studies** |
| TNF_{α} | Adults | 11.5 ng/L | 55 | 66 | *(30)* |
| | Neonates | 12-20 ng/L | 67/79/88 | 43/71/86 | *(29, 61 63)* |
| IL-6 | Adults | 50-200 ng/L | 51/67/86 | 53/65/79 | *(30,35,36,66)* |
| | Neonates | 10-160 ng/L | 71/84/100 | 43/71/96 | *(26, 29, 61, 63, 70-72)* |
| IL-1ra | Children | NA^{b} | 33 | 89 | *(85)* |
| | Neonates | 10.9 µg/L | 93 | 92 | *(70)* |
| IL-8 | Adults | 30-340 ng/L | 57/63/68 | 57/76/93 | *(30, 35, 85)* |
| | Neonates | 50 ng/L | 92 | 70 | (61) |
| CRP | Adults | 4-150 mg/L | 35/69/89 | 18/61/81 | *(30, 35, 36,38, 46 66, 88, 89)* |
| | Neonates | 1-23 mg/L | 43/65/96 | 80/90/100 | *(22, 26, 63, 70, 89, 90)* |
| PCT | Adults | 0.4-81 µg/L | 65/81/97 | 48/73/94 | *(30,35-38, 43,* 46, 66, *88, 89, 122, 123)* |
| | Neonates | 1.0-61 µg/L | 77/85/99 | 62/83/91 | *(22, 72, 89, 90)* |
| ^{a}Values listed are for differentiating infected individuals from uninfected controls rather than from healthy individuals. | | | | | |
| Sensitivities and specificities Ested are minimum, mean [In bold], and maximum percentages. | | | | | |
| ^{b} NA. not avalable. | | | | | |

This overview may be found in EP 2 060 920 A1.

These data show that even in humans, where septic disease patterns are extensively investigated, sensitivity and specificity of current markers can (even as mean values) come down to as low as 33% and 66% respectively, not to mention the inhomogeneity of presently published data.

The use of anti-trypsin for the diagnosis of different types of sepsis and for differential diagnosis of different types of sepsis has been elucidated by Soares et. al (Revista Brasileira de Terapia Intensiva (2007) 14-22); Ren Yan et. al (Journal of Proteome Research (2007) 2812-2821); and US 2009/104605 (Siuzdak Gary [US] et. al (2009)). Nevertheless, the simple evidence of anti-trypsin as shown, without a certain cut-off value or evidence of specific fragments of the markers, is not sensitive and/or specific enough to provide a reliable method for the risk stratification and/or differential diagnosis of sepsis.

These data show that there is definitely a need for new diagnostic markers with improved clinical characteristics. Therefore, the diagnosis of sepsis, especially early diagnosis of sepsis, is still a great need in clinical medicine. An optimum diagnosis should reveal persons with a risk of developing sepsis or persons being at an early stage of sepsis. Therefore, there is a need for clinical diagnostic markers which can reliably detect and diagnose an infectious induced systemic inflammation which may further develop into sepsis as early as possible before the onset of organ dysfunction, i.e., the onset of sepsis. Hence, these new clinical diagnostic markers need to reliably differentiate an infectious induced systemic inflammation from a non-infectious induced systemic inflammation, which may also include or lead to life-threatening organ dysfunction and may occur in polytraumatized patients but crucially without underlying infection. So far there has been a lack of reliable clinical diagnostic markers which achieve this differentiation.

It is therefore an object of the present invention to provide a suitable method for diagnosing sepsis or prior stages of sepsis, i.e., a method which is sensitive and/or specific to an infectious induced systemic inflammation in contrast to a non- infectious induced systemic inflammation.

The endogenous balance between inhibitory active forms of the major human SERPIN alpha-1-antitrypsin (A1AT) and its target-proteinases (primarily human neutrophil elastase, HNE) is a crucial factor determining whether local inflammation processes will result in larger connective tissue damage. Complex formation of A1AT with serine proteases does not only cause inactivation of both substrates but also cleavage of the SERPIN at the reactive site centered at Met³⁸²-Ser. However, several non-target enzymes are also able to cleave A1AT in the RCL (reactive center loop) region causing functional loss of the primary regulator towards human serine proteinases and thus increase the risk for the development of lung emphysema and other acute or chronic inflammatory conditions which are known pathophysiological consequences among A1AT deficient individuals.

During the process of A1AT cleavage by certain enzymes (with or without complex formation), carboxyl-terminal peptides of A1AT (CAAPs) of various lengths can emerge. In addition to HNE which provokes the formation of C36 when complexed to A1AT, many other enzymes have been identified exhibiting efficient A1AT degradation activity with simultaneous generation of CAAPs. Those enzymes include either endogenous human proteases (mainly matrix-metalloproteinases), proteases found in microbes causing infections or proteases that act as allergens for human allergic disorders. However, CAAPs have been detected predominantly by using *in vitro* incubation experiments with purified A1AT. A few CAAPs have also been identified *in vivo* in in human tissue and liquids such as urine, bronchoalveolar lavage fluid, gingival crevicular fluid, spleen and bile, lung tissue carotid artery tissue, placenta and nipple aspiration fluids. Some CAAPs were proposed to serve as putative biomarker for glomular kidney diseases, pulmonary fibrosis, gingivitis and carotid artery stenosis.

However, C42 ("CAAP48/47") is the hitherto only CAAP detected in human blood. In a previous study, C42 has been shown to function as inflammatory marker because C42 levels were significantly elevated in patients with severe sepsis compared with patients with systemic inflammatory response syndrome (SIRS, according to the now outdated Sepsis-2 definition as provided below) or HIV, respectively. Due to the higher concentrations of C42 found in the sepsis compared to SIRS group, a crucial role of microbes frequently triggering septic shock can be assumed. This is supported by the fact that extracellular microbial proteases, identified *in vitro* as CAAP-generating enzymes, serve as virulence factors for *Staphylococcus aureus, Pseudomonas aeruginosa, Serratia marcescens* and *Candida albicans.*

In addition to the role of C42 as sepsis biomarker, CAAPs reveal immunomodulary functions as well and thus might contribute themselves to the inflammatory process. Among the multiplicity of A1AT cleavage fragments, C36 and C42 are the best studied CAAPs regarding their inflammatory immune modulating functions. They act, for example, as chemoattractant for phagocytes and monocytes, activators of human monocytes and neutrophils *in vitro,* neutrophil extracellular trap inhibitory substances and alter hepatic functions and gene expression *in vitro* and *in vivo.* From a pathophysiological perspective, CAAPs seem to be highly relevant as diagnostic tools for the identification of a variety of inflammatory diseases.

A variety of cleavage products of the major plasma protease inhibitor alpha-1-antitrypsin (CAAPs) have been observed *in vitro* by incubation of alpha-1-antitrypsin with proteases as well as *in vivo* in several human tissues. With the exception of C42 ("CAAP48/47"), none of those CAAPs have been detected in human blood yet. Due to the vital role of CAAPs not only during inflammation and infection but possibly also, for example, in the course and pathogenesis of allergic diseases a rapid screening method using readily available biomaterials such as blood-derived specimens is needed.

This invention discloses a LC-MS/MS method as a diagnostic tool determining CAAP patterns. The invention comprises a fast, multiplexed LC-MS/MS method for the simultaneous quantification of CAAPs in biomaterial samples obtained from patients.

### Definitions

Herein, "systemic inflammation" is the result of release of pro-inflammatory cytokines from immune-related cells and the chronic activation of the innate immune system, which is not limited to a specific site of inflammation but occurs throughout the system. Systemic inflammation may be either induced by infection or may have non-infectious causes. As said above sepsis only arises from infectious induced systemic inflammation. Hence, infectious induced systemic inflammation can be understood as an early stage of sepsis if onset of sepsis is not prevented by timely clinical intervention.

According to sepsis-3 definition, "sepsis" is understood as life-threatening organ dysfunction (organ failure) caused by a dysregulated host response to infection, i.e., the sepsis results from an infectious-induced systemic inflammation, when organ failure occurs due to the host response to the underlying infection. In this respect organ dysfunction is understood as acute change by at least 2 points in SOFA-score (sequential organ failure assessment), as determination of the SOFA-score is time consuming an alternative q-SOFA-score (quick SOFA) has been established, which includes at least 2 of the following clinical criteria altered mentation, respiratory rate of 22/min or greater, or systolic blood pressure of 100 mm Hg or less. "Septic shock" is accordingly understood as a subset of sepsis, wherein patients are clinically identified by vasopressor requirement to maintain a mean arterial pressure of 65 mm Hg or greater and serum lactate level greater than 2 mmol/L (> 18 mg/dL) in the absence of hypovolemia (Singer et al. in JAMA, 2016; doi: 10.1001/jama.2016.0287). The person skilled in the art is also aware that sepsis may be further differentiated into several distinct endotypes which differ mechanistically, so far up to 5 distinct endotypes based on distinct mechanisms have been described, wherein each mechanism shows a distinct pattern of gene expression (Baghela et al.; EBioMedicine, 2021; doi.org/10.1016/j.ebiom.2021.103776).

"Septic complications" are further symptoms or clinical consequences which are caused by sepsis or occur in combination with sepsis.

"Early stages of sepsis" or "initial sepsis" is herein synonymous with infectious induced systemic inflammation in particular before onset of organ dysfunction, i.e., onset of sepsis.

In the following when reference is made to either of the terms "systemic inflammation", "early stages of sepsis", "initial sepsis", "sepsis", "septic shock" or "complication of sepsis" the other terms as well as sepsis endotypes are *mutatis mutandis* always also implied as and if applicable.

For the sake of completeness and to properly refer to data sets prior of the revised sepsis-3 definition, the now outdated definition for sepsis according to sepsis-2 definition is also provided. According to sepsis-2 definition, sepsis was understood as a systemic inflammatory syndrome according to the following definition which is caused by an underlying infection. "Systemic Inflammatory Response Syndrome" (or "SIRS") is defined as the systemic inflammatory response to a variety of severe clinical insults manifested by two or more of the following conditions: 1) temperature > 38°C or < 36°C; 2) heart rate > 90 beats per minute; 3) respiratory rate > 20 breaths per minute or PaCO2 < 32 mm Hg; and 4) white blood cell count > 12,000/cu mm; < 4,000/cu mm, or > 10% immature (band) forms. When SIRS is the result of a confirmed infectious process, it is termed sepsis (Bone RC, Balk RA, Cerra FB, Dellinger RP, Fein AM, Knaus WA, et al. Definitions for sepsis and organ failure and guidelines for the use of innovative therapies in sepsis. The ACCP/SCCM Consensus Conference Committee. American College of Chest Physicians/Society of Critical Care Medicine. Chest. 1992 Jun.;101(6):1644-1655.)

Herein, "alpha-1-antitrypsin" is also referred to as antitrypsin and "ATT". Alpha-1-Antitrypsin or α-1-antitrypsin (A1AT) is a protease inhibitor belonging to the serpin superfamily. It is generally known as serum trypsin inhibitor. Alpha-1-antitrypsin is also referred to as alpha-1-proteinase inhibitor (A1PI) because it inhibits a wide variety of proteases. It protects tissues from enzymes of inflammatory cells, especially neutrophil elastase, and has a reference range in blood of 1.5 - 3.5 g/l, but the concentration can rise manyfold upon acute inflammation. In its absence, neutrophil elastase is free to break down elastin, which contributes to the elasticity of the lungs, resulting in respiratory complications such as emphysema, or COPD (chronic obstructive pulmonary disease) in adults and cirrhosis in adults or children.

In the context of the present invention, the terms "threshold", "threshold value", "cut-off" and "cut-off value" are used synonymously.

The term "correlating," as used herein in reference to the use of diagnostic and prognostic markers, refers to comparing the presence or amount of the marker(s) in a patient to its presence or amount in persons known to suffer from, or known to be at risk of, a given condition; or in persons known to be free of a given condition. As discussed above, a marker level in a patient sample can be compared to a level known to be associated with a specific diagnosis. The sample's marker level is said to have been correlated with a diagnosis; that is, the skilled artisan can use the marker level to determine whether the patient suffers from a specific type of diagnosis and respond accordingly. Alternatively, the sample's marker level can be compared to a marker level known to be associated with a good outcome (e.g., the absence of disease, etc.). In preferred embodiments, profiles of marker levels are correlated to a global probability or a particular outcome.

A "prognosis" refers to assignment of a probability that a given course or outcome will occur. This is often determined by examining one or more "prognostic indicators". These are markers, the presence or amount of which in a patient (or a sample obtained from the patient) signal a probability that a given course or outcome will occur. For example, when one or more prognostic indicators reach a sufficiently high level in samples obtained from such patients, the level may signal that the patient is at an increased probability for eventually advancing into end-stage renal disease (ESRD), i.e. the patient has an increased probability of being a "progressor".

### Detailed description of the invention

The present invention solves the above described short-coming of the prior art in reliable diagnosis of sepsis or early/prior stages of sepsis by reliably differentiating infectious induced systemic inflammation from non-infectious induced systemic inflammation. This is achieved by disclosing a mass-spectrometry based method of detecting a selection of peptides which are formed e.g., by protease mediated proteolysis or direct transcription of those peptides (Matmala et al.; PLOS ONE (2017); doi: 10.1371/journal.pone.0170533). The detected patterns and profiles of these peptides are then clinical diagnostic markers which are indicative of the underlying disease. The profiles of this peptide panel can be also further applied to other differential diagnoses such as differentiating (i) non-infectious systemic inflammation, (ii) infectious systemic inflammation, (iii) infection, (iv) initial sepsis, (v) different endogenous sepsis phenotypes and (v) septic shock.

Nine full-length C-terminal peptides were selected and synthesized compromising each CAAP amino acid lengths (from C45 to C22) in WT and SNP variant rs1303, respectively. To be able to quantitatively measure this peptide panel a multiplex MRM-based method was established and validated for human plasma (EDTA and lithium-heparin) and serum.

### Selection of target compounds

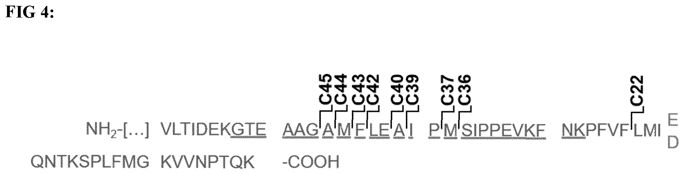

Within the scientific literature a variety of cleavage sites of A1AT are known. Among the variety of C-terminal peptides of A1AT (CAAPs) a panel of nine full-length CAAPs was selected (Figure 4) according to their experimental evidence. The skilled person will understand that the method of the invention may also be applied to fragments that were only predicted as well as intermediate fragments in the C-terminal region of A1AT (such as VIRIP), which have not been tested.

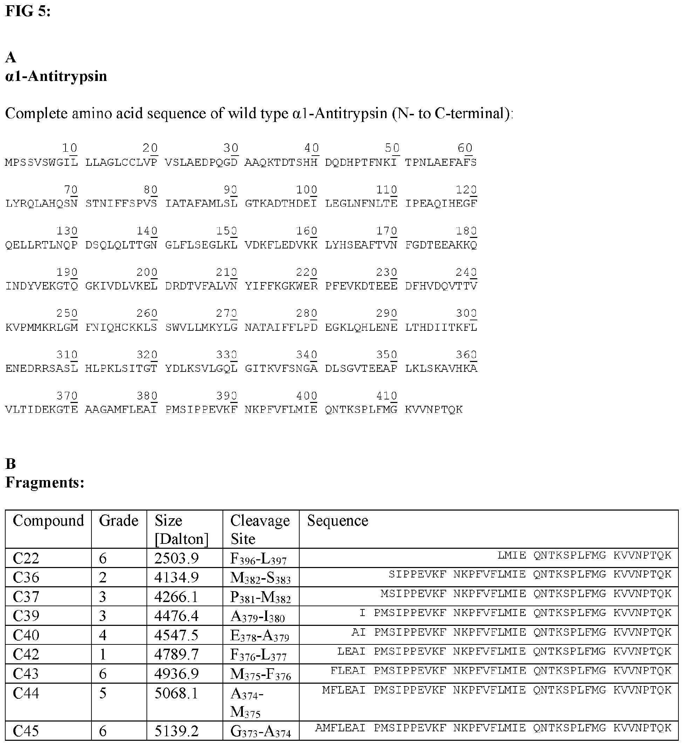

The fragments are those shown in Figures 4 and Figure 5.

Due to a minor allele frequency for the single nucleotide polymorphisms (SNP) of A1AT (rs1303, 28%) within the investigated C-terminal amino acid sequence, the final CAAP panel consists of eighteen analytes (wild type [WT] and SNP for each CAAP length). Authentic CAAPs and internal standards were obtained from sb-PEPTIDE (SmartBioscience SAS, Saint Egrève, FRANCE) as lyophilized peptides in aliquots. C22, C37 and C42 containing isotopic labelled lysine (¹³C¹⁵N) were used as internal standards (IS) for quantification (C22^{IS}, C37^{IS}, C42^{IS}). The skilled person will understand that the method of the invention is not exclusively limited to detecting the peptides of this selection, the method of the invention can be adjusted to detect differently selected peptide panels comprising degradation product peptides of A1AT, this includes but is not limited to peptides obtained from other A1AT SNPs.

In one embodiment of the invention, it comprises a method for quantitatively measuring a concentration of a peptide panel in a biological matrix by mass spectrometry detection, wherein multiplex multiple reaction monitoring (MRM) scanning is applied.

The method of the invention comprises the steps a)-d):
a) sample collection,
b) sample preparation,
c) detection by mass spectrometry,
d) statistical analyses and quantification.

After collection of the sample from the patient sample preparation comprises mixing sample with internal standard and a sample solvent and centrifugation and optionally storage at -80°C.

In one embodiment of the invention the peptide panel is detected by mass spectrometry which comprises LC-MS/MS analysis comprising the steps a)-c):
a) mixing sample with internal standard and a sample solvent,
b) chromatographic separation,
c) mass spectrometry.

In one embodiment of the invention the sample solvent is selected from the group comprising cooled methanol.

In one embodiment of the invention mass spectrometry involves chromatographic separation which may be performed by application of a gradient of solvents.

In some embodiments of the invention the solvents for gradient chromatography are selected from the group comprising formic acid in water/acetonitrile and water/methanol, wherein a first solvent consists of 0.01-1 % formic acid in pure water and a second solvent consists of 0.01-1 % formic acid in pure acetonitrile or pure methanol, preferably the first and second solvents contain 0.05-0.5 % formic acid and most preferably 0.1 % formic acid. The person skilled in the art will be aware that other equivalent combinations and compositions of solvent are available and may be applied without changing the method of the invention. Such embodiments are also part of the present invention. Further solvents may be selected from but are not limited to the group consisting of trifluoroacetic acid in water or acetonitrile, ethanol, propanol, isopropanol, triethylamine, tetrahydrofuran, dichloromethane/methane and nitromethane. The person skilled in the art will further understand that water and organic solvents may be used in different mixtures with varying mixing ratios.

The solvents for gradient chromatography may be mixed in different mixing ratios, wherein the first solvent is applied in 0-100 % and the second solvent is applied in 0-100 %. Mixing ratios and flow rates of solvents may be varied stepwise during gradient chromatography.

The method of the invention can be used to detect CAAPs in different samples or biological matrices which are human tissues selected from the group comprising spleen and placenta and /or exhaled breath/exhaled breath condensate(EBC) and/or human liquids selected from the group comprising whole blood, blood and blood fractions, serum, plasma, lymphatic fluid, urine, tears, saliva, sputum, bile, bronchoalveolar lavage fluid, pleural effusions, cerebrospinal fluid (CSF), nipple aspiration fluid, umbilical cord blood and punctate samples taken from, e.g., ascites or pleura or an extract of any of the aforementioned samples.

In one embodiment of the invention plasma samples may be treated with anticoagulants, e.g. EDTA or lithium-heparin.

The method of the invention enables detection of a peptide panel comprising one or more proteolytic degradation product peptides of a protein.

In one embodiment of the invention the peptides are associated with a condition selected from the group comprising infection, inflammation, systemic inflammation, septic shock, sepsis, severe sepsis, cancer, pulmonary fibrosis, kidney diseases, allergies.

In one embodiment of the invention the peptides are sepsis biomarkers.

In one embodiment of the invention the peptides are degradation products of major human SERPIN alpha-1-antitrypsin (A1AT) protein.

In one embodiment of the invention the protein is proteolytically degraded by one or more human and/or microbial (fungal, bacterial) and/or viral proteases selected from the group comprising matrix-metalloproteinases (MMPs), serine proteases, cysteine proteases, metalloproteases, cathepsins, semi-alkine proteinase, preferably selected from the group comprising human neutrophil elastase (HNE), matrix-metalloproteases (MMP) 1, 3, 7-9, 11-13, 25 and 26, cathepsin L, prostate-specific antigen, mesotrypsin, high temperature requirement A1, trypsin, Glycyl endopeptidase, Adamalysin II, Thermolysin, Periodontain, Aureolysin, Papain, Glutamyl endopeptidase, Staphopain A, Pseudolysin and der p1 allergen as well as viral serine proteases.

In one embodiment of the invention mean accuracies are within ±20% of lower limit of quantification.

In one embodiment of the invention mean precisions are within ±20% of lower limit of quantification.

In one embodiment of the invention carry-over is less than ±5% of peak areas of the peptide's lower limit of quantification.

In one embodiment the method of the invention includes a method for the diagnosis, prediction or risk stratification for mortality or disease outcome of a subject that has or is suspected to have sepsis, comprising the steps of (a) - (c):
a) detection and quantification of a peptide panel by LC-MS/MS according to the invention,
b) wherein the measured concentration of peptides is correlated with an increased risk of mortality or poor disease outcome or diagnosis and/or prognosis and, wherein
c) said increased risk of mortality or poor disease outcome or diagnosis and/or prognosis is given if the concentration of peptides is below a certain cut-off value and/or the concentration of peptides is above a certain cut-off value.

In another embodiment the present invention relates to a method for the diagnosis, prediction or risk stratification for mortality or disease outcome of a subject that has or is suspected to have sepsis, comprising
- determining the level of CAAPs (from C45 to C22) in a sample taken from said subject by a multiplex MRM-based method,
- wherein the level of CAAPs is correlated with an increased risk of mortality or poor disease outcome or diagnosis and/or prognosis, and,
- wherein said increased risk of mortality or poor disease outcome or diagnosis and/or prognosis is given if the level of CAAPs is below a certain cut-off value and/or the level of CAAPs is above a certain cut-off value.

The method according to the invention also involves comparing the level of marker for the individual/patient/subject to diagnosed with a predetermined value. The predetermined value can take a variety of forms. It can be single cut-off value: This can be for instance a median or mean or the 75^{th}, 90^{th}, 95^{th} or 99^{th} percentile of a reference population. This can be for instance also an "optimal" cut-off value. The optimal cut-off value for a given marker is the value where the product of diagnostic sensitivity and specificity is maximal for this marker. Diagnostic sensitivity is the relative fraction of patients, carrying the disease or the risk for developing the disease (depending on the diagnostic or prognostic question to be answered in any particular case), which are correctly recognized as such by a marker ("true positives"), and the diagnostic specificity is the relative fraction of patients, not carrying the disease or the risk for developing the disease (depending on the diagnostic or prognostic question to be answered in any particular case), which are recognized as such by a marker ("true negatives"). This can by a cut-off value optimized for a maximal negative predictive value or maximal positive predictive value, depending on clinical or economical needs. Thereby optimizing specificity and sensitivity.

Thus, one might adopt the cut-off value depending on whether it is considered more appropriate to identify most of the subjects at risk at the expense of also identifying "false positives", or whether it is considered more appropriate to identify mainly the subjects at high risk at the expense of missing several subjects at moderate risk.

The predetermined value can be established based upon comparative groups, such as where the risk in one defined group is double the risk in another defined group. It can be a range, for example, where the tested population is divided equally (or unequally) into groups, such as a low-risk group, a medium-risk group and a high-risk group, or into quartiles, the lowest quartile being individuals with the lowest risk and the highest quartile being individuals with the highest risk.

The predetermined value can vary among particular reference populations selected, depending on their habits, ethnicity, genetics etc. Accordingly, the predetermined values selected may take into account the category in which an individual falls. Appropriate ranges and categories can be selected with no more than routine experimentation by those of ordinary skill in the art.

In certain embodiments, particular thresholds for one or more markers in a panel are not relied upon to determine if a profile of marker levels obtained from a subject are indicative of a particular diagnosis/prognosis. Rather, the present invention may utilize an evaluation of a marker panel "profile" as a unitary whole. A particular "fingerprint" pattern of changes in such a panel of markers may, in effect, act as a specific diagnostic or prognostic indicator. As discussed herein, that pattern of changes may be obtained from a single sample, or from temporal changes in one or more members of the panel (or a panel response value). A panel herein refers to a set of markers. Herein, at least one of the CAAP markers as described herein may be combined with determination of further clinical markers for sepsis, which include but are not limited to A1AT, A1AT-CAAP ratio, CRP, PCT, IL-6, pro-ADM and/or TTR. The person skilled in the art is aware of a number of further potential clinical markers, which are hereby also included in possible marker panels according to this invention.

In another embodiment of the invention at least two of the CAAPs of this invention either on their own or in combination with further clinical markers as mentioned above may form a marker panel with a specific marker profile. As the composition and ratios of the present human, microbial and viral proteases will vary depending on the underlying infection the resulting profile of the marker panel will accordingly vary as well. Hence, the profile of the marker panel can be used to diagnose the specific infectious agent or agents causing the underlying infection. This means that from the specific pattern or profile of the elevated CAAPs as detected by the present method either on their own or in combination with further clinical markers the specific infectious agent or class of agent such as but not limited to fungi, bacteria or viruses can be inferred.

A panel response value can be derived by various methods. One example is Cox proportional hazards analysis. Another example is optimizing ROC curves: This can be achieved by plotting ROC curves for the sensitivity of a particular panel of markers versus 1-(specificity) for the panel at various cut-offs.

In these methods, a profile of marker measurements from a subject is considered together to provide a global probability (expressed either as a numeric score or as a percentage risk) of a diagnosis or prognosis. In such embodiments, an increase in a certain subset of markers may be sufficient to indicate a particular diagnosis/prognosis in one patient, while an increase in a different subset of markers may be sufficient to indicate the same or a different diagnosis/prognosis in another patient. Weighting factors may also be applied to one or more markers in a panel, for example, when a marker is of particularly high utility in identifying a particular diagnosis/prognosis, it may be weighted so that at a given level it alone is sufficient to signal a positive result. Likewise, a weighting factor may provide that no given level of a particular marker is sufficient to signal a positive result, but only signals a result when another marker also contributes to the analysis.

In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/- 5% of a given measurement.

The skilled artisan will understand that associating a diagnostic or prognostic indicator, with a diagnosis or with a prognostic risk of a future clinical outcome is a statistical analysis. For example, a marker level of greater than X may signal that a patient is more likely to suffer from an adverse outcome than patients with a level less than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations and determining a confidence interval and/or a p value. See, e.g., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

In yet other embodiments, multiple determinations of diagnostic or prognostic markers can be made, and a temporal change in the marker can be used to determine a diagnosis or prognosis. For example, a marker concentration in a subject sample may be determined at an initial time, and again at a second time from a second subject sample. In such embodiments, an increase in the marker from the initial time to the second time may be indicative of a particular diagnosis, or a particular prognosis. Likewise, a decrease in the marker from the initial time to the second time may be indicative of a particular diagnosis, or a particular prognosis.

The term "sample" as used herein refers to either a tissue sample or a sample of bodily fluid obtained for the purpose of diagnosis, prognosis, or evaluation of a subject of interest, such as a patient. Preferred test samples include blood, serum, plasma, cerebrospinal fluid, urine, saliva, sputum, and pleural effusions. In addition, one of skill in the art would realize that some test samples would be more readily analyzed following a fractionation or purification procedure, for example, separation of whole blood into serum or plasma components.

Thus, in a preferred embodiment of the method according to the invention said sample is selected from human tissues selected from the group comprising spleen and placenta and/or exhaled breath/exhaled breath condensate (EBC) and/or human liquids selected from the group comprising whole blood, blood and blood fractions, serum, plasma, lymphatic fluid, urine, tears, saliva, sputum, bile, bronchoalveolar lavage fluid, pleural effusions, cerebrospinal fluid (CSF), nipple aspiration fluid, umbilical cord blood and punctate samples taken from, e.g., ascites or pleura or an extract of any of the aforementioned samples.

In a preferred embodiment of the invention the level of CAAPs is correlated with the prediction or risk stratification for mortality or disease outcome by a method which is selected from the following group of alternatives:
- correlation with respect to the median of the level of CAAPs in an ensemble of predetermined samples,
- correlation with respect to quantiles of the level of CAAPs in an ensemble of predetermined samples, and
- correlation with a mathematical model, such as for example Cox Regression.

More preferably, the level of CAAPs have a cut-off value for the prediction or risk stratification for mortality or disease outcome, wherein CAAPs levels below the cut-off value indicate a healthy subject and CAAPs levels above the cut-off value indicate a disease state.

The cut-off value of CAAP level for C36 peptide is between about 0.147 and about 0.197 µM, preferably between about 0.152 and about 0.172 µM, more preferably about 0.162 µM, and may deviate depending on the patient analysed by about 5%, 10% or even 20%. This cut-off value was determined in ETDA plasma and may differ according to sample or blood-derived specimen type (e.g. urine or serum).

According to the invention the level of CAAP C36 peptide which is associated with diagnosis of sepsis or an increased risk for mortality is
- above the range between about 0.147 and about 0.197 µM,
- preferably above the range between about 0.152 and about 0.172 µM,
- more preferably above about 0.162 µM.

These ranges may deviate depending on the patient analysed by about 5%, 10% or even 20%. These ranges were determined in ETDA plasma and may differ according to sample or blood-derived specimen type (e.g. urine or serum).

The cut-off value of CAAP level for C37 peptide is between about 0.034 and about 0.080 µM, preferably between about 0.045 and about 0.069 µM, more preferably about 0.048 µM, and may deviate depending on the patient analysed by about 5%, 10% or even 20%. This cut-off value was determined in ETDA plasma and thus cut-off values may differ depending on the sample type used (e.g. urine or serum). Therefore, the skilled person will understand, that it may be necessary to establish distinct sets of cut-off values for each sample type.

According to the invention the level of CAAP C37 peptide which is associated with diagnosis of sepsis or an increased risk for mortality is
- above the range between about 0.034 and about 0.080 µM,
- preferably above the range between about 0.045 and about 0.069 µM,
- more preferably above about 0.048 µM.

These ranges may deviate depending on the patient analysed by about 5%, 10% or even 20%. These ranges were determined in ETDA plasma and thus cut-off values may differ depending on the sample type used (e.g. urine or serum). Therefore, the skilled person will understand, that it may be necessary to establish distinct sets of cut-off values for each sample type.

The cut-off value of CAAP level for C40 peptide is between about 0.017 and about 0.053 µM, preferably between about 0.018 and about 0.031 µM, more preferably about 0.020 µM, and may deviate depending on the patient analysed by about 5%, 10% or even 20%. This cut-off value was determined in ETDA plasma and thus cut-off values may differ depending on the sample type used (e.g. urine or serum). Therefore, the skilled person will understand, that it may be necessary to establish distinct sets of cut-off values for each sample type.

According to the invention the level of CAAP C40 peptide which is associated with diagnosis of sepsis or an increased risk for mortality is
- above the range between about 0.017 and about 0.053 µM,
- preferably above the range between about 0.018 and about 0.031 µM,
- more preferably above about 0.020 µM.

These ranges may deviate depending on the patient analysed by about 5%, 10% or even 20%. These ranges were determined in ETDA plasma and thus cut-off values may differ depending on the sample type used (e.g. urine or serum). Therefore, the skilled person will understand, that it may be necessary to establish distinct sets of cut-off values for each sample type.

The cut-off value of CAAP level for C42 peptide is between about 0.126 and about 0.169 µM, preferably between about 0.130 and about 0.148 µM, more preferably about 0.137 µM, and may deviate depending on the patient analysed by about 5%, 10% or even 20%. This cut-off value was determined in ETDA plasma and thus cut-off values may differ depending on the sample type used (e.g. urine or serum). Therefore, the skilled person will understand, that it may be necessary to establish distinct sets of cut-off values for each sample type.

According to the invention the level of CAAP C42 peptide which is associated with diagnosis of sepsis or an increased risk for mortality is
- above the range between about 0.126 and about 0.169 µM,
- preferably above the range between about 0.130 and about 0.148 µM,
- more preferably above about 0.137 µM.

These ranges may deviate depending on the patient analysed by about 5%, 10% or even 20%. These ranges were determined in ETDA plasma and thus cut-off values may differ depending on the sample type used (e.g. urine or serum). Therefore, the skilled person will understand, that it may be necessary to establish distinct sets of cut-off values for each sample type.

The invention relates to a method, wherein a sample is taken at one or more of the following time points, when the subject is first admitted to a medical institution or in the ambulance, when the subject is in the emergency room, when the subject is in the intensive care unit, before treatment, after initiation of treatment, 24 hours after initiation of treatment, 48 hours after initiation of treatment, and/or 72 hours after initiation of treatment or in the course of the diseases at regular intervals.

The subject is under a condition selected from the group comprising infection, sepsis and septic shock.

In addition to prediction or risk stratification for mortality or disease outcome of said subject the determination of the level of CAAPs in a sample taken from said subject, is used herein to differentially diagnose whether said subject is likely to have non-infectious induced systemic inflammation, infectious-induced systemic inflammation, initial sepsis, severe sepsis/septic shock or to differentially diagnose sepsis endotypes.

The said samples are human tissues selected from the group comprising spleen and placenta and/or exhaled breath/exhaled breath condensate (EBC) and/or human liquids selected from the group comprising whole blood, blood and blood fractions, serum, plasma, lymphatic fluid, urine, tears, saliva, sputum, bile, bronchoalveolar lavage fluid, pleural effusions, cerebrospinal fluid (CSF), nipple aspiration fluid, umbilical cord blood and punctate samples taken from, e.g., ascites or pleura or an extract of any of the aforementioned samples.

The invention relates to a method of medical decision making for individual patient therapy related to the severity of the disease by monitoring therapy response in a subject with sepsis or a sepsis like disease to a certain drug, comprising the steps of,
- taking at least two samples from said subject at various time points selected from the group of,
   i. before initiation of therapy and/or
   ii. after initiation of therapy, and/or
   iii. at one or more further time point
- determining the level of CAAPs in said sample taken from said subject,
- associating the levels of CAAPs in said samples taken from said subject with a positive or a negative response to said certain drug.

Said positive response is given if the level of CAAPs fragments decreases during drug treatment.

The invention preferably relates to a method of medical decision making for individual patient therapy in a subject related to the severity of the disease, comprising the steps of,
- taking at least one sample from said subject at various possible time points selected from the group of,
   i. before initiation of therapy and/or
   ii. after initiation of therapy, and/or
   iii. at one or more further time point
- determining level of CAAPs in said sample taken from said subject, and
- associating the level(s) of CAAPs in said samples taken from said subject with the need for a certain therapy.

Said correlation of the level of CAAPs to (i) initial sepsis, (ii) sepsis or (iii) septic shock is given if the level of CAAPs is above a certain cut-off value.

The invention preferably relates to a method for differentially diagnosing a disease in a subject, wherein the diseases are selected from the group of (i) non-infectious systemic inflammation, (ii) infectious systemic inflammation, (iii) infection, (iv) initial sepsis, (v) different endogenous sepsis phenotypes and (vi) septic shock, said method comprising the steps of:
(1) determining the level of CAAPs in a blood sample taken from said subject, and
(2) correlating the level of CAAPs to (i) non-infectious systemic inflammation, (ii) infectious systemic inflammation, (iii) infection, (iv) initial sepsis, (v) different endogenous sepsis phenotypes and (vi) septic shock,
(3) wherein said correlation of the level of CAAPs to (i) non-infectious systemic inflammation, (ii) infectious systemic inflammation, (iii) infection, (iv) initial sepsis, (v) different endogenous sepsis phenotypes and (vi) septic shock is given if the level of CAAPs is above or below a certain cut-off value.

The skilled person will clearly understand that for differential diagnosis further sets of cut-off values for each separate possible diagnosis (i) non-infectious systemic inflammation, (ii) infectious systemic inflammation, (iii) infection, (iv) initial sepsis, (v) different endogenous sepsis phenotypes and (vi) septic shock will have to be determined. The skilled person is aware of the necessary steps for determining these further cut-off values.

In another aspect the invention relates to a method for differentially diagnosing the causal infection of sepsis or septic shock in a subject, wherein the causal infections are selected from the group of (i) viral infection, (ii) bacterial infection, (iii) fungal infection or (iv) other microbial infections, said method comprising the steps of:
(1) determining the level of CAAPs in a blood sample taken from said subject, and
(2) correlating the level of CAAPs to (i) viral infection, (ii) bacterial infection, (iii) fungal infection or (iv) other microbial infections,
(3) wherein said correlation of the level of CAAPs to (i) viral infection, (ii) bacterial infection, (iii) fungal infection or (iv) other microbial infections is given if the level of CAAPs is above or below a certain cut-off value.

The skilled person will clearly understand that for differential diagnosis further sets of cut-off values for each separate possible diagnosis (i) viral infection, (ii) bacterial infection, (iii) fungal infection or (iv) other microbial infections will have to be determined. The skilled person is aware of the necessary steps for determining these further cut-off values.

The skilled person will further understand that for all described methods of detection of this invention the cut-off values will also depend on the tested sample material and will have to be established for each material individually. The skilled person is aware of the necessary steps for determining these further cut-off values.

Another aspect of the invention is a kit for diagnosis of sepsis or predicting the prognosis in a patient with sepsis, comprising:
a) standard peptides of the peptide panel,
b) internal standards and
c) reference data and concentrations of the analytes for healthy patients and under disease conditions,
d) standard data showing the correlation between the concentration of peptides contained in samples and diagnosis and/or prognosis and optionally
e) a manual.

The kit enables the user to quantify the levels of CAAPs LC-MS/MS. These are then compared to standard data sets.

Preferably the following protein is detected (SEQ ID NO. 1). As outlined above and below, in sepsis the ratio of the full length protein to the fragments shown below changes.

**Table 2: α1-Antitrypsin**

| |
|---|
| Complete amino acid sequence (N- to C-terminal; SEQ ID NO. 1) |
| 10 20 30 40 50 60  |
| SEQ ID NO. 20 is the protein above without the leader and has the following sequence. |
| EDPQGD AAQKTDTSHH DQDHPTFNKI TPNLAEFAFS LYRQLAHQSN STNIFFSPVS IATAFAMLSL GTKADTHDEI LEGLNFNLTE IPEAQIHEGF QELLRTLNQP DSQLQLTTGN GLFLSEGLKL VDKFLEDVKK LYHSEAFTVN FGDTEEAKKQ INDYVEKGTQ GKIVDLVKEL DRDTVFALVN YIFFKGKWER PFEVKDTEEE DFHVDQVTTV KVPMMKRLGM FNIQHCKKLS SWVLLMKYLG NATAIFFLPD EGKLQHLENE LTHDIITKFL ENEDRRSASL HLPKLSITGT YDLKSVLGQL GITKVFSNGA DLSGVTEEAP LKLSKAVHKA VLTIDEKGTE AAGAMFLEAI PMSIPPEVKF NKPFVFLMIE QNTKSPLFMG KVVNPTQK |

**Table 3: Sequences of the analytes:**

| SEQ ID NO. | Analyt | Grade | Size^{#} | Cleavage Site | Sequence |
|---|---|---|---|---|---|
| 2 | C22^{WT} | 6 | 2502.33 | F₃₉₆-L₃₉₇ | LMIE QNTKSPLFMG KVVNPTQK |
| 3 | C22^{SNP} | 6 | 2488.32 | F₃₉₆-L₃₉₇ | LMID QNTKSPLFMG KVVNPTQK |
| 4 | C36^{WT} | 2 | 4132.23 | M₃₈₂-S₃₈₃ | SIPPEVKF NKPFVFLMIE QNTKSPLFMG KVVNPTQK |
| 5 | C36^{SNP} | 2 | 4118.21 | M₃₈₂-S₃₈₃ | SIPPEVKF NKPFVFLMID QNTKSPLFMG KVVNPTQK |
| 6 | C37^{WT} | 3 | 4263.27 | P₃₈₁-M₃₈₂ | MSIPPEVKF NKPFVFLMIE QNTKSPLFMG KVVNPTQK |
| 7 | C37^{SNP} | 3 | 4249.25 | P₃₈₁-M₃₈₂ | MSIPPEVKF NKPFVFLMID QNTKSPLFMG KVVNPTQK |
| 8 | C39^{WT} | 3 | 4473.40 | A₃₇₉-I₃₈₀ | I PMSIPPEVKF NKPFVFLMIE QNTKSPLFMG KVVNPTQK |
| 9 | C39^{SNP} | 3 | 4459.39 | A₃₇₉-I₃₈₀ | I PMSIPPEVKF NKPFVFLMID QNTKSPLFMG KVVNPTQK |
| 10 | C40^{WT} | 4 | 4544.44 | E₃₇₈-A₃₇₉ | AI PMSIPPEVKF NKPFVFLMIE QNTKSPLFMG KVVNPTQK |
| 11 | C40^{SNP} | 4 | 4530.42 | E₃₇₈-A₃₇₉ | AI PMSIPPEVKF NKPFVFLMID QNTKSPLFMG KVVNPTQK |
| 12 | C42^{WT} | 1 | 4786.57 | F₃₇₆-L₃₇₇ | LEAI PMSIPPEVKF NKPFVFLMIE QNTKSPLFMG KVVNPTQK |
| 13 | C42^{SNP} | 1 | 4772.55 | F₃₇₆-L₃₇₇ | LEAI PMSIPPEVKF NKPFVFLMID QNTKSPLFMG KVVNPTQK |
| 14 | C43^{WT} | 6 | 4933.64 | M₃₇₅-F₃₇₆ | FLEAI PMSIPPEVKF NKPFVFLMIE QNTKSPLFMG KVVNPTQK |
| 15 | C43^{SNP} | 6 | 4919.62 | M₃₇₅-F₃₇₆ | FLEAI PMSIPPEVKF NKPFVFLMID QNTKSPLFMG KVVNPTQK |
| 16 | C44^{WT} | 5 | 5064.68 | A₃₇₄-M₃₇₅ | MFLEAI PMSIPPEVKF NKPFVFLMIE QNTKSPLFMG KVVNPTQK |
| 17 | C44^{SNP} | 5 | 5050.66 | A₃₇₄-M₃₇₅ | MFLEAI PMSIPPEVKF NKPFVFLMID QNTKSPLFMG KVVNPTQK |
| 18 | C45^{WT} | 6 | 5135.71 | G₃₇₃-A₃₇₄ | AMFLEAI PMSIPPEVKF NKPFVFLMIE QNTKSPLFMG KVVNPTQK |
| 19 | C45^{SNP} | 6 | 5121.70 | G₃₇₃-A₃₇₄ | AMFLEAI PMSIPPEVKF NKPFVFLMID QNTKSPLFMG KVVNPTQK |

| | | | | | |
|---|---|---|---|---|---|
| ^{#} theoretical monoisotopic mass (g/mol) | | | | | |

Table 3 shows fragments of the full length protein. The table also classifies the fragments in more or less preferable for the diagnosis/prognosis of sepsis (see "grade": 1 being very preferable and 6 being only preferable).

SEQ ID NO. 2 and 3 may be very preferably used.

SEQ ID NO. 4 and 5 may be preferably used.

SEQ ID NO. 6 and 7 may be used.

SEQ ID NO. 1 and 8-19 may also be used.

In the methods outlined above one or more of the fragments may be detected. Any combination of fragments above is hence disclosed and claimed without having to write them all down which the skilled person is able to do.

Moreover, the invention also relates to the use of a polypeptide according to SEQ ID NO. 1-19 and/or 20 in a method for diagnosing sepsis, preferably in a method as outlined above.

The invention further relates to a reference standard for A1AT fragments produced by a method comprising the step of incubating A1AT with HNE or MMP-7 under conditions suitable for cleavage. The advantage of this kind of reference standard over an artificial, synthetic reference standard is a closer similarity to in vivo occurring A1AT fragments. The reference may be used to determine molecular weight of in vivo molecules identified, to determine the concentration or as any other reference.

The references cited herein are incorporated by reference in their entirety. The invention has been shown and described with references to preferred embodiments thereof. The invention will be understood by those skilled in the art that various changes in form and details may be made therein without departing from the scope of the invention as defined by the claims.

### Figure captions

Figure 1: Comparison of CAAP levels in EDTA plasma, lithium-heparin plasma and serum. Relative changes (compared to EDTA plasma) were calculated for each donor individually (n=6) before mean accuracy (%) and CV (%) were calculated.
Figure 2: CAAPs abundance in healthy individuals (n=6) and septic patients (n=36, subgroup A). Data are based on measurements of EDTA plasma samples, which were obtained from patients within the first three days after onset of sepsis. Statistical analyse was conducted using Mann-Whitney Rank Sum Test. ^{∗∗∗} P<0.001, n.d. not detected, n.s. not significant

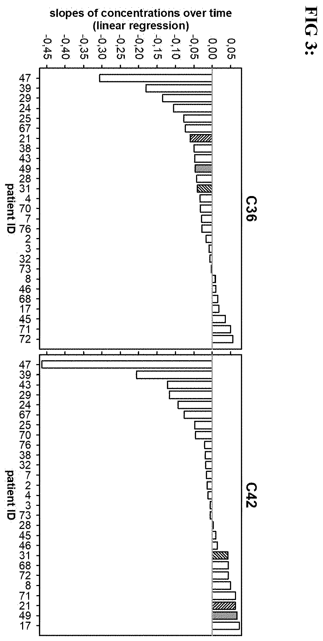

Figure 3: Development of C36 und C42 concentrations in septic patients. To obtain trends over time, linear regression analyses were conducted for patients with at least three sample collected during their stay at the ICU (subgroup B, n=27).
Figure 4: Cleavage sites of selected CAAPs within the C-terminal amino acid sequence of A1AT. CAAPs are named after their respective amino acid lengths after cleavage. The sequence includes single nucleotide polymorphism rs1303 in the CAAP sequence at position 376 (E>D³⁷⁶). Underlined: 25 residue reactive centre loop of A1AT (UniProtKB).
Figure 5: Sequences of the full-length wild type α1-Antitrypsin and the sequences of the C-terminal degradation peptides.
Figure 6: representative chromatograms of CAAPs and internal standards.
Figure 7: results of specificity testing for individual CAAPs.
Figure 8: linear responses of CAAPs in different blood-derived matrices.
Figure 9: results of stability testing of analytes.

### Examples

### Example 1: Sample preparation

Lyophilized authentic peptide standards were dissolved and diluted in a 70 g/L Albumin (from human serum, lyophilized, ≥99%, Sigma-Aldrich) in PBS (Sigma-Aldrich, St. Louis, MO, USA) solution. Internal standards C37^{IS} and C42^{IS} were dissolved in Milli-Q water (Millipore, Brussels, Belgium) containing 0.1% (*v*/*v*) formic acid (≥95%, Sigma-Aldrich) whereas C22^{IS} was only dissolved in ultrapure water. Mixtures of CAAPs used as calibration standards (calibrants) and quality control samples (QCs) were prepared as stock solution to a final concentration of 14 µM, respectively, (expect C40^{SNP} which was 5 µM) with subsequent serial dilutions. Calibrants and QCs were obtained from separate serial dilutions. Internal standard solution was prepared by adding equal volumes of each IS yielding a final concentration of 0.8 µM, respectively. All samples used for method development and validation were prepared and stored in 0.5 mL Protein LoBind Tubes (Eppendorf AG, Hamburg, Germany) in aliquots at -80°C until use.

For LC-MS/MS analyses, 10 µL of internal standard solution was added to 35 µL of calibrant, QC or study sample and gently mixed by pipetting. This mixture was then briefly centrifuged (10 sec; 16,000*g*; +10 °C; model 5415R, Eppendorf AG) before 90 µL of cooled methanol (≥ 99.9 %, Carl Roth, Karlsruhe, Germany) was added and briefly vortexed subsequently. After centrifugation (10 min; 16,000*g*; 10 °C) 100 µL of supernatant was transferred into 2 mL brown glass autosampler vials using 200 µL glass inserts (Wicom, Heppenheim, Germany).

### Example 2: LC-MS/MS analyses

For chromatographic separation, a Shimadzu HPLC system (Duisburg, Germany) was used which is equipped with a binary pump (LC20AB), a thermostatic autosampler (SIL20AC) and a thermostatic column compartment (CTO20AC). 5 µL sample was injected from the autosampler (maintained at 10 °C) and loaded on the UHPLC column (bioZen 3.6 µM Intact C4, 2.1 ^{×} 100 mm, Phenomenex, Aschaffenburg, Germany). The column oven was maintained at 45 °C; solvent A was 0.1% formic acid in ultrapure water (*v*/*v*) and solvent B was 0.1% formic acid in LC-MS Grade acetonitrile (*v*/*v*, ≥ 99.95 %, Carl Roth). A step gradient was applied as follows: 5-25% B from 0-1 min., 25-46% B from 1-6 min., 46-85%B from 6-6.5 min., 85% B from 6.5-8.5 min., 85-5% B from 8.5-9.0 min. and 5% B from 9-12 min. The flow rate was 350 µL/min from start to 6.5 min. and 10.6 to 12 min., respectively, and 600 µL/min from 7.0 to 10.5 min.

For mass spectrometry detection, a Triple Quad 5500+ (AB SCIEX, Framingham, MA) in multiple reaction monitoring (MRM) scan type was used with Q1 at low and Q3 at unit resolution. The instrument was equipped with a Turbo V^{™} ion source and operated in positive electrospray ionization mode with source parameters as follows: curtain gas = 35, collision gas = 8, ionSpray voltage = 5500, temperature = 700 °C, nebulization gas (GS1) = 70, drying gas (GS2) = 70.

**Table 4 Compound-dependent parameters for the detection and quantification of CAAPs.**

| **Compound** (mass^{#}) | | **RT-window** [min] | **Precursor** | | **Product** [*m*/*z*] | **DP** [V] | **CE** IVÌ | **CXP** [V] | **IS** |
|---|---|---|---|---|---|---|---|---|---|
| | | | ***m*/*z*** | **charge** | | | | | |
| **C22**^{WT} (2502.33) | Quantifier | 3.3-3.5 | 626.8 | [M+4H]⁴⁺ | 754.1 | 110 | 25 | 7 | C22^{IS} |
| | Qualifier | | 626.7 | | 217.3 | 125 | 30 | 20 | |
| **C22^{SNP}** (2488.32) | Quantifier | 3.3-3.5 | 623.3 | | 749.4 | 110 | 25 | 7 | |
| | Qualifier | | 623.3 | | 217.3 | 125 | 30 | 20 | |
| **C36^{WT}** (4132.23) | Quantifier | 4.2-4.7 | 689.84 | [M+6H]⁶⁺ | 787.8 | 105 | 20 | 6 | C37^{IS} |
| | Qualifier | | 689.84 | | 173.4 | 125 | 50 | 15 | |
| **C36^{SNP}** (4118.21) | Quantifier | 4.1-4.6 | 687.49 | | 785 | 105 | 20 | 6 | |
| | Qualifier | | 687.49 | | 173.4 | 125 | 20 | 15 | |
| **C37^{WT}** (4263.27) | Quantifier | 4.3-4.8 | 711.74 | [M+6H]⁶⁺ | 787.8 | 105 | 30 | 6 | |
| | Qualifier | | 854.09 | [M+5H]⁵⁺ | 984.8 | 125 | 40 | 20 | |
| **C37^{SNP}** (4249.25) | Quantifier | 4.3-4.8 | 709.5 | [M+6H]⁶⁺ | 785.1 | 105 | 30 | 6 | |
| | Qualifier | | 851.26 | [M+5H]⁵⁺ | 981.1 | 125 | 40 | 20 | |
| **C39^{WT}** (4473.40) | Quantifier | 4.4-5.0 | 747.06 | [M+6H]⁶⁺ | 728.2 | 105 | 30 | 6 | |
| | Qualifier | | 896.05 | [M+5H]⁵⁺ | 984.5 | 125 | 40 | 15 | |
| **C39^{SNP}** (4459.39) | Quantifier | 4.3-4.9 | 744.63 | [M+6H]⁶⁺ | 785.1 | 105 | 35 | 6 | |
| | Qualifier | | 744.63 | | 129.2 | 125 | 40 | 15 | |
| **C40^{WT}** (4544.44) | Quantifier | 4.4-5.0 | 758.25 | [M+6H]⁶⁺ | 728.2 | 105 | 20 | 6 | C42^{IS} |
| | Qualifier | | 910.38 | [M+5H]⁵⁺ | 873.6 | 125 | 30 | 20 | |
| **C40^{SNP}** (4530.42) | Quantifier | 4.3-4.9 | 756.45 | [M+6H]⁶⁺ | 725.9 | 105 | 20 | 6 | |
| | Qualifier | | 907.51 | [M+5H]⁵⁺ | 870.8 | 125 | 30 | 10 | |
| **C42^{WT}** (4786.57) | Quantifier | 4.6-5.2 | 799.2 | [M+6H]⁶⁺ | 873.6 | 105 | 25 | 6 | |
| | Qualifier | | 958.6 | [M+5H]⁵⁺ | 1091.9 | 125 | 40 | 20 | |
| **C42^{SNP}** (4772.55) | Quantifier | 4.5-5.1 | 796.75 | [M+6H]⁶⁺ | 870.7 | 105 | 25 | 6 | |
| | Qualifier | | 956 | [M+5H]⁵⁺ | 1088.4 | 125 | 40 | 15 | |
| **C43^{WT}** (4933.64) | Quantifier | 4.9-5.5 | 823.65 | [M+6H]⁶⁺ | 873.4 | 105 | 25 | 6 | |
| | Qualifier | | 988.28 | [M+5H]⁵⁺ | 1091.9 | 125 | 40 | 15 | |
| **C43^{SNP}** (4919.62) | Quantifier | 4.8-5.4 | 821.3 | [M+6H]⁶⁺ | 870.8 | 105 | 25 | 6 | |
| | Qualifier | | 985.4 | [M+5H]⁵⁺ | 1088.2 | 120 | 40 | 15 | |
| **C44^{WT}** (5064.68) | Quantifier | 5.0-5.6 | 845.35 | [M+6H]⁶⁺ | 873.9 | 105 | 35 | 6 | |
| | Qualifier | | 1014.5 | [M+5H]⁵⁺ | 1091.9 | 125 | 40 | 15 | |
| **C44^{SNP}** (5050.66) | Quantifier | 5.0-5.5 | 843.08 | [M+6H]⁶⁺ | 870.7 | 100 | 35 | 6 | |
| | Qualifier | | 1011.59 | [M+5H]⁵⁺ | 1088.1 | 125 | 40 | 15 | |
| **C45^{WT}** (5135.71) | Quantifier | 5.1-5.7 | 857.59 | [M+6H]⁶⁺ | 873.3 | 100 | 35 | 6 | |
| | Qualifier | | 1028.58 | [M+5H]⁵⁺ | 1091.9 | 125 | 40 | 15 | |
| **C45^{SNP}** (5121.70) | Quantifier | 5.1-5.6 | 855.19 | [M+6H]⁶⁺ | 871.0 | 100 | 35 | 6 | |
| | Qualifier | | 1025.85 | [M+5H]⁵⁺ | 1088.1 | 120 | 40 | 15 | |
| **C22^{IS}** (2516.37) | IS | 3.3-3.5 | 630.06 | [M+4H]⁴⁺ | 756.4 | 110 | 25 | 7 | - |
| **C37^{IS}** (4277.30) | IS | 4.3-4.8 | 714.01 | [M+6H]⁶⁺ | 790.6 | 105 | 30 | 6 | |
| **C42^{IS}** (4807.62) | IS | 4.7-5.2 | 802.72 | [M+6H]⁶⁺ | 876.3 | 105 | 25 | 6 | |

| | | | | | | | | | |
|---|---|---|---|---|---|---|---|---|---|
| ^{#} theoretical monoisotopic mass (g/mol). DP = declustering potential, RT = retention time, CE = collision energy, CXP = collision cell exit potential, SNP = single nucleotide polymorphism, IS = internal standard | | | | | | | | | |

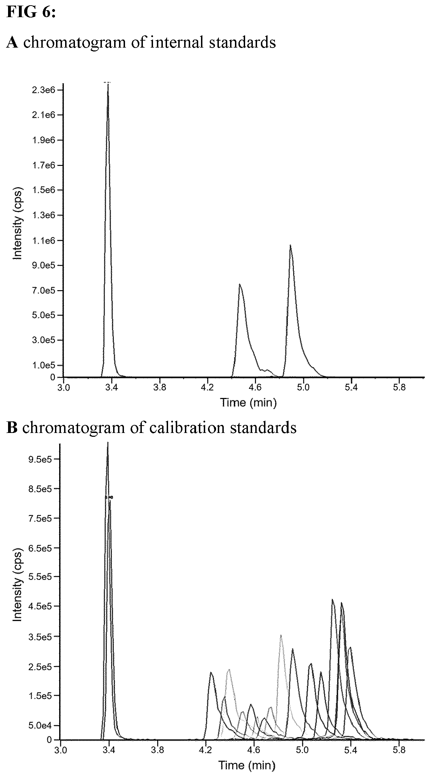

Two transitions were monitored for each compound (quantifier, qualifier) and one for the internal standards using an intensity threshold of 50 cps. Corresponding compound-dependent parameters were optimized for each transition (table 4) whereas entrance potential was 10 V for all transitions. Dwell time was 35 msec for all quantifier and 10 msec for all qualifier. A representative chromatogram is shown in **Figure 6**.

### Example 3: Method validation

### Linearity, working range, carry-over and specificity

Nine calibrants were used in total ranging from 0.01 µM to 1.5 µM thereby covering more than two orders of magnitude in concentration. In eight independent analytical runs, mean linear regression coefficients of each calibration curve from 0.01 to 1.5 µM was R > 0.99 for each compound (CV < 0.4%, n = 8, respectively) providing sufficient **linearity** throughout the entire calibration range. Working ranges for each of the CAAPs were set according to concentrations in healthy and critically ill patients and were optimized during validation procedure (table 5).

**Table 5: Working ranges, QC sample concentrations and results of carry-over experiments. Carry-over is determined as the ratio of peak areas measured in blank albumin sample to peak areas of respective LLOQs (%).**

| analyte | working range (no. of calibrants) | concentrations of QCs used for validation | carry-over |
|---|---|---|---|
| C36^{WT} | 0.025-1.5 µM (8) | QC2 (0.025 µM, LLOQ)/ QC3 (0.3 µM)/ QC4 (0.9 µM) | 4% |
| C36^{SNP} | | | 2% |
| C42^{WT} | | | 2% |
| C42^{SNP} | | | 1% |
| C37^{WT} | 0.01-1 µM (8) | QC1 (0.01 µM, LLOQ) QC3 (0.3 µM) QC4 (0.9 µM) | 0% |
| C37^{SNP} | | | 0% |
| C22^{WT} | 0.01-0.5 µM (7) | QC1 (0.01 µM, LLOQ) QC2 (0.025 µM) QC3 (0.3 µM) | 0% |
| C22^{SNP} | | | 1% |
| C39^{WT} | | | 0% |
| C39^{SNP} | | | 0% |
| C40^{WT} | | | 0% |
| C43^{WT} | | | 0% |
| C43^{SNP} | | | 2% |
| C44^{WT} | | | 0% |
| C44^{SNP} | | | 2% |
| C45^{WT} | | | 0% |
| C45^{SNP} | | | 0% |
| C40^{SNP} | 0.0036-0.1785 µM (7) | QC1 (0.0036 µM, LLOQ) QC2 (0.089 µM) QC3 (0.1073 µM) | 0% |

**Carry-over** was less than 5% of peak areas compared to respective LLOQ samples for all CAAPs and are thus in line with official recommendations (below 20% for analytes, table 5). In none of the blank albumin samples measured directly after respective ULOQ samples internal standards were detected.

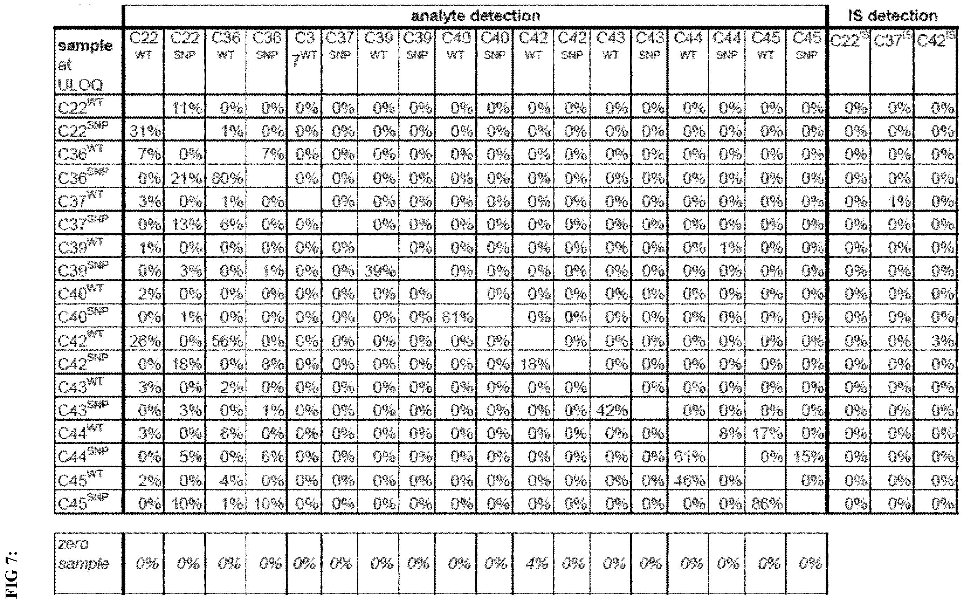

No interferences (test of **specificity)** above the LLOQ were detected (**Figure 7**). We detected a few WT-variants above 20% of the LLOQ in the presence of respective SNP-variants. There was further no interference greater than 5% between CAAPs and internal standard detection (**Figure 7**).

### Accuracy and precision

Mean accuracies and precisions (within-run and between-run) were within recommended ranges (maximum ±15% and ±20% for LLOQ, respectively) (table 6).

**Table 6: Accuracy and precision of repeated measurements.**

| Analyte | | quality control | **Within-run** (technical replicates, n = 5) | | **Between-run** (independent runs, n = 5) | |
|---|---|---|---|---|---|---|
| | | | Accuracy (mean, %) | Precision (mean CV, %) | Accuracy (mean, %) | Precision (mean CV, %) |
| C36^{WT} | | QC2/ QC3/ QC4 | 110/ 111/ 107 | 5/ 2/ 1 | 113/ 108/ 106 | 2/ 3/ 2 |
| C36^{SNP} | | | 113/ 108/ 104 | 1/ 3/ 1 | 113/ 107/ 104 | 1/ 2/ 2 |
| C42^{WT} | | | 109/ 107/ 104 | 2/ 2/ 3 | 110/ 106/ 105 | 3/ 2/ 3 |
| C42^{SNP} | | | 107/ 106/ 104 | 3/ 2/ 3 | 109/ 106/ 105 | 2/ 2/ 3 |
| C37^{WT} | | QC1/ QC3/ QC4 | 117/ 108/ 104 | 4/ 2/ 2 | 121/ 108/ 104 | 6/ 3/ 2 |
| C37^{SNP} | | | 113/ 107/ 105 | 5/ 2/ 3 | 117/ 105/ 103 | 2/ 2/ 3 |
| C22^{WT} | | QC1/ QC2/ QC3 | 99/ 100/ 100 | 2/ 3/ 3 | 103/ 105/ 102 | 4/ 4/ 2 |
| C22^{SNP} | | | 98/ 101/ 102 | 3/2/4 | 101/ 105/ 102 | 7/ 3/ 3 |
| C39^{WT} | | | 120/ 114/ 109 | 5/ 1/ 1 | 117/ 119/ 106 | 2/4/4 |
| C39^{SNP} | | | 111/ 111/ 103 | 3/ 4/ 2 | 117/ 110/ 103 | 7/ 3/ 1 |
| C40^{WT} | | | 115/ 112/ 106 | 3/ 4/ 2 | 117/ 112/ 106 | 2/ 3/ 2 |
| C40^{SNP} | | | 112/ 108/ 104 | 8/ 6/ 2 | 105/ 103/ 101 | 5/ 2/ 2 |
| C43^{WT} | | | 110/ 108/ 99 | 3/ 2/ 2 | 117/ 111/ 101 | 4/ 4/ 2 |
| C43^{SNP} | | | 109/ 107/ 100 | 5/ 2/ 2 | 117/ 112/ 103 | 5/ 3/ 3 |
| C44^{WT} | | | 117/ 113/ 103 | 1/ 2/ 2 | 118/ 112/ 103 | 10/ 7/ 4 |
| C44^{SNP} | | | 112/ 107/ 103 | 2/ 2/ 2 | 113/ 111/ 102 | 9/ 9/ 4 |
| C45^{WT} | | | 117/ 116/ 104 | 2/ 2/ 2 | 115/ 111/ 104 | 8/ 8/ 4 |
| C45^{SNP} | | | 112/ 111/ 102 | 5/ 1/ 2 | 111/ 111/ 102 | 6/5/4 |

### Selectivity and investigation of matrix effects

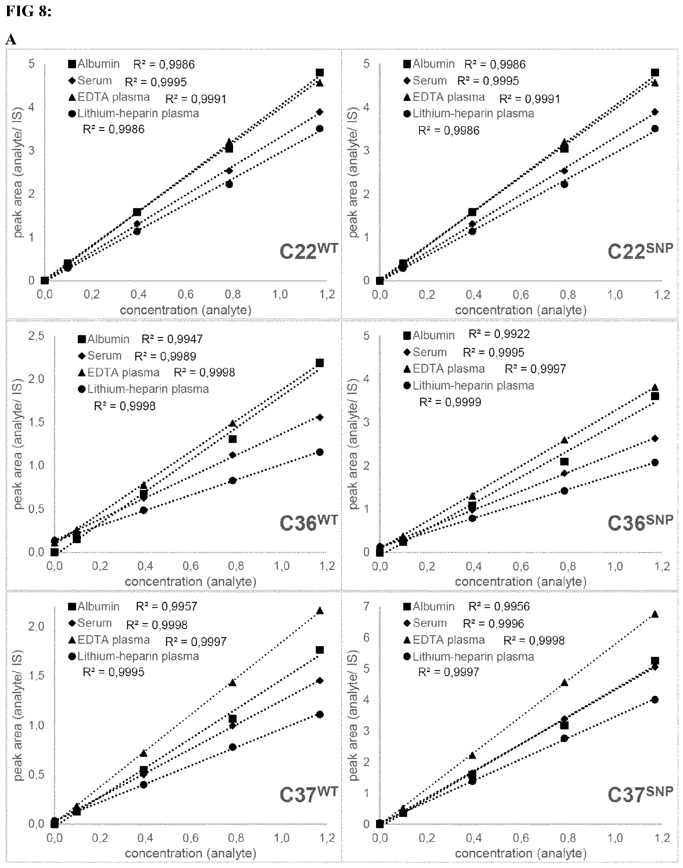

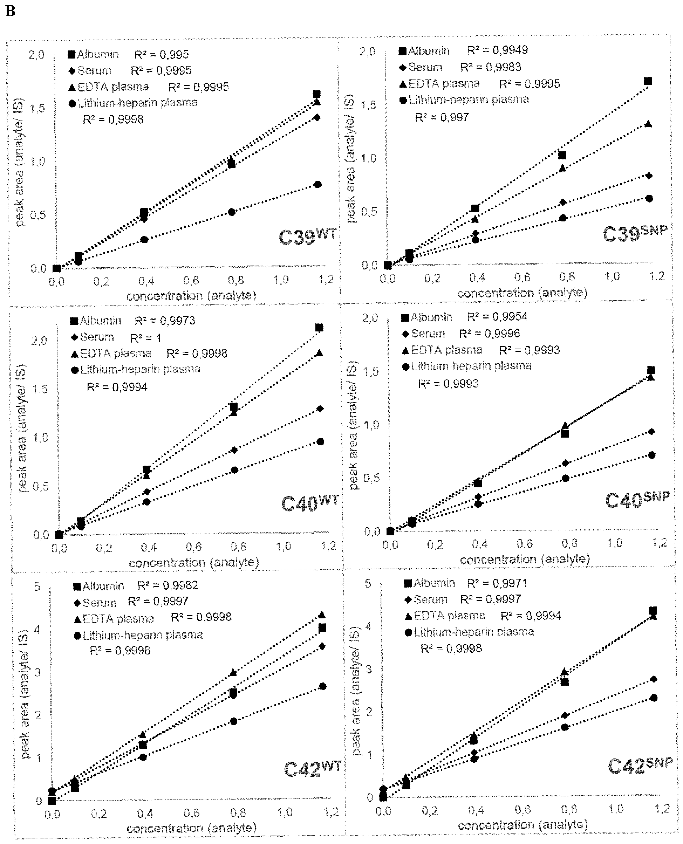

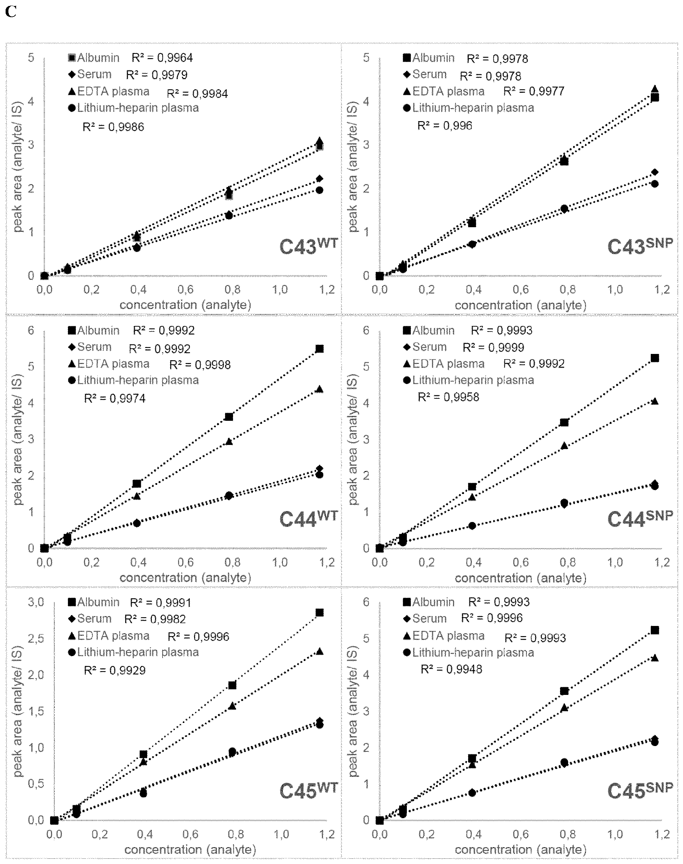

Selectivity was determined in blank albumin samples and results revealed that no peaks (analyte or internal standards) were detected. Serial dilutions of CAAPs standard in pool (human) matrices verified that the responses of concentrations in albumin, serum and plasma (EDTA and lithium-heparin) are linear, respectively (**Figure 8**). Due to the unavailability of analyte-free biological matrix, matrix effects were investigated by determining recovery rates and precisions of each compound in different human whole blood derived specimens which were compared to zero albumin samples with equal amount of CAAPs added (using area ratios instead of back-calculated concentrations). Most of the analytes spiked into EDTA plasma were within ±20% of recovery compared to zero albumin sample (table 7). Precisions were ≤10% between six individually analyzed human donors suggesting that matrix effects are low in EDTA plasma. Recovery rates of CAAPs in lithium-heparin as well as in serum were clearly lower than those in EDTA plasma revealing that effects of these matrices are considerably high. However, most of the CAAP concentrations in serum were consistently back-calculated among the six measured healthy individuals (precision ≤ 20%).

**Table 7: Recovery rates of spiked CAAP concentrations in different biological matrices. Accuracies and precisions are shown as mean of six voluntary donors. H: high CAAPs concentration; L: low CAAPs concentration.**

| | **EDTA plasma** | | | | **lithium-heparin plasma** | | | | **Serum** | | | |
|---|---|---|---|---|---|---|---|---|---|---|---|---|
| | accuracy (mean, n = 6) | | precision (n = 6) | | accuracy (mean, n = 6) | | precision (n = 6) | | accuracy (mean, n = 6) | | precision (n = 6) | |
| | H | L | H | L | H | L | H | L | H | L | H | L |
| **C22^{WT}** | 98% | 99% | 4% | 1% | 46% | 46% | 10% | 13% | 58% | 59% | 8% | 13% |
| **C22^{SNP}** | 98% | 99% | 3% | 2% | 46% | 46% | 11% | 13% | 58% | 59% | 8% | 14% |
| **C36^{WT}** | 99% | 91% | 2% | 5% | 25% | 20% | 21% | 68% | 49% | 50% | 10% | 18% |
| **C36^{SNP}** | 105% | 109% | 4% | 4% | 28% | 29% | 24% | 38% | 52% | 53% | 12% | 14% |
| **C37^{WT}** | 125% | 126% | 5% | 6% | 40% | 42% | 14% | 14% | 66% | 65% | 5% | 9% |
| **C37^{SNP}** | 129% | 132% | 4% | 3% | 51% | 55% | 11% | 17% | 80% | 80% | 6% | 9% |
| **C39^{WT}** | 84% | 78% | 6% | 8% | 31% | 33% | 16% | 21% | 47% | 44% | 11% | 17% |
| **C39^{SNP}** | 83% | 80% | 9% | 10% | 19% | 21% | 25% | 30% | 30% | 29% | 21% | 27% |
| **C40^{WT}** | 117% | 105% | 5% | 8% | 28% | 28% | 19% | 22% | 47% | 43% | 12% | 14% |
| **C40^{SNP}** | 102% | 102% | 5% | 2% | 27% | 29% | 17% | 21% | 43% | 44% | 13% | 17% |
| **C42^{WT}** | 100% | 97% | 2% | 7% | 36% | 34% | 13% | 31% | 52% | 52% | 9% | 17% |
| **C42^{SNP}** | 99% | 103% | 4% | 7% | 28% | 28% | 21% | 31% | 39% | 39% | 17% | 21% |
| **C43^{WT}** | 119% | 121% | 5% | 6% | 44% | 45% | 12% | 13% | 57% | 55% | 5% | 7% |
| **C43^{SNP}** | 113% | 116% | 3% | 6% | 33% | 34% | 14% | 15% | 38% | 40% | 11% | 12% |
| **C44^{WT}** | 81% | 79% | 8% | 5% | 18% | 18% | 19% | 28% | 25% | 23% | 11% | 20% |
| **C44^{SNP}** | 82% | 80% | 8% | 6% | 15% | 17% | 25% | 39% | 21% | 22% | 15% | 23% |
| **C45^{WT}** | 77% | 72% | 7% | 10% | 24% | 20% | 19% | 23% | 35% | 27% | 7% | 14% |
| **C45^{SNP}** | 88% | 88% | 8% | 5% | 20% | 24% | 21% | 22% | 30% | 30% | 12% | 16% |

To clarify reasons for high variations of peak area ratios of CAAPs in lithium-heparin plasma, variations of internal standards were additionally analyzed (table 8).

**Table 8: Mean peak areas and precisions of internal standards in different biological matrices. Different CAAP concentrations were spiked into each of the individual matrices: 0 = pure albumin; H: high CAAPs concentration; L: low CAAPs concentration.**

| | | **Peak Area** | | | | | |
|---|---|---|---|---|---|---|---|
| | No. of values | C42^{IS} | | C37^{IS} | | C22^{IS} | |
| | | mean | CV (%) | mean | CV (%) | mean | CV (%) |
| **Albumin (H, L)** | n = 4 | 9.2^{∗}10⁶ | 2.7% | 7.0^{∗}10⁶ | 3.0% | 7.9^{∗}10⁶ | 1.2% |
| **EDTA (0, H, L)** | n = 34 | 7.7^{∗}10⁶ | 6.4% | 5.7^{∗}10⁶ | 9.6% | 7.6^{∗}10⁶ | 5.4% |
| **Li-Hep (0, H, L)** | n = 35 | 6.7^{∗}10⁶ | 18.5% | 4.7^{∗}10⁶ | 19.4% | 7.3^{∗}10⁶ | 14.1% |
| **Serum (0, H, L)** | = 33 | 6.0^{∗}10⁶ | 8.5% | 4.2^{∗}10⁶ | 10.2% | 7.0^{∗}10⁶ | 6.4% |

High variations in recovery of CAAPs in lithium-heparin plasma derived most likely from inconsistent responses of all three internal standards in lithium-heparin plasma.

### Stability

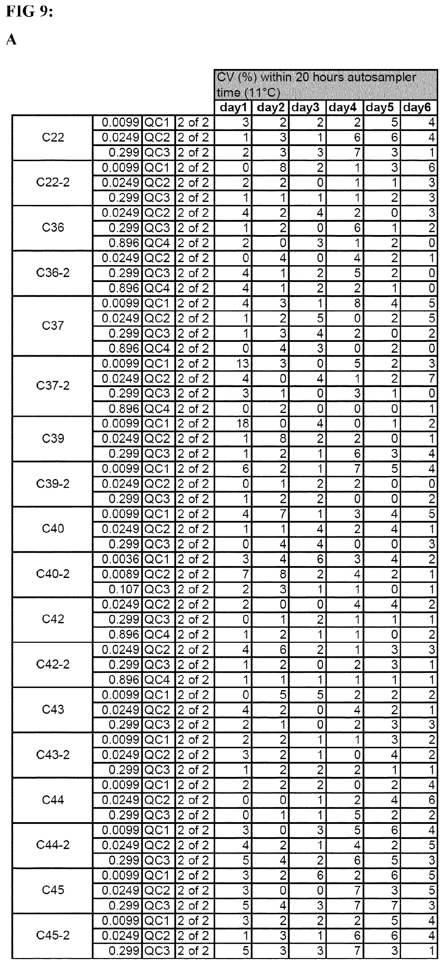

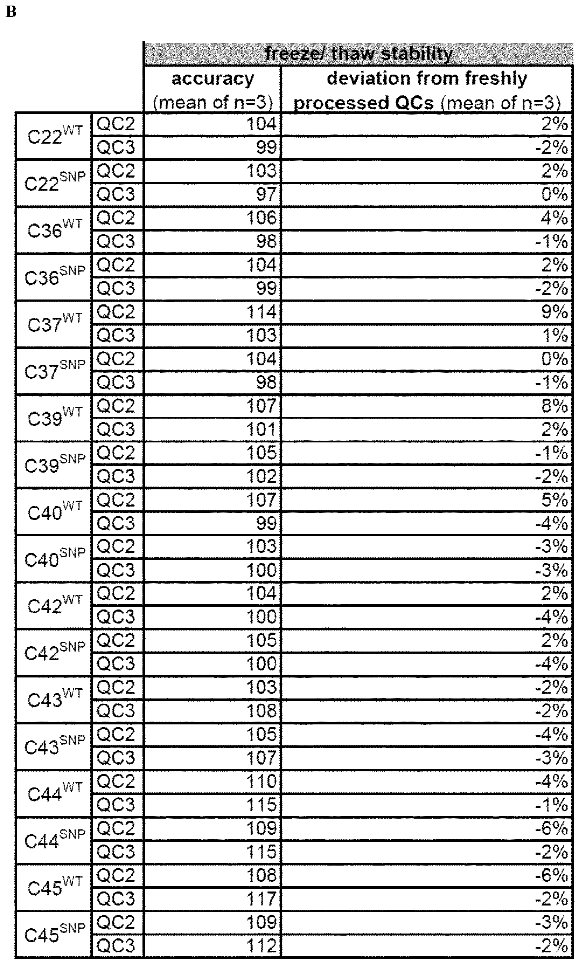

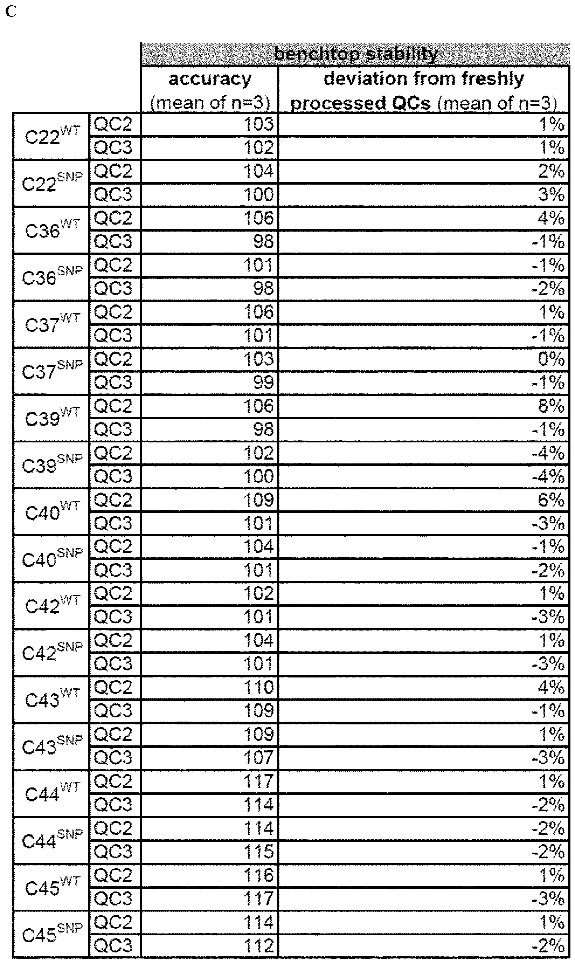

Stability of analytes in processed samples was determined at six independent days for a time span of 20 hours in the autosampler. Deviations of standing samples to freshly measured samples were below 19% at the LLOQ and below 9% for other QC levels for each analyte, respectively (**Figure 9**). Stability of analytes in biological matrix was determined by consecutive cycles of freezing and thawing as well as extended intervals at room temperature from thawing to processing (approximately one hour). Deviations of either freeze/ thaw treatment or extended benchtop time at room temperature compared to freshly processed QCs did not exceed ±10% (CV) and mean accuracies were within 98-117, respectively (**Figure 9**).

### Example 4: Quantification of CAAPs in healthy humans and comparison between different whole blood-derived specimens

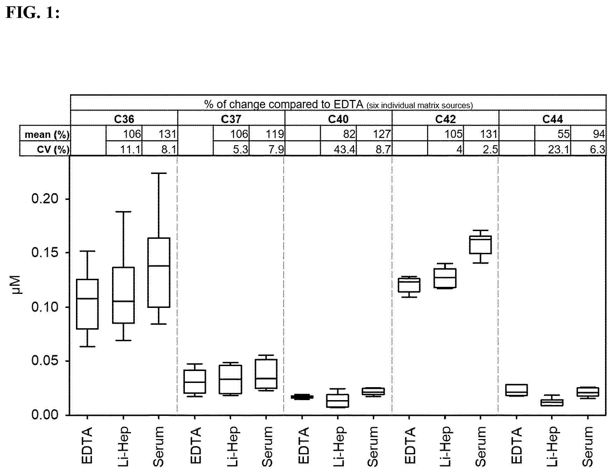

Using the current screening MRM-based method we were able to quantify concentrations at baseline levels from healthy donors (n=6) for five out of nine CAAPs investigated (namely C36, C37, C40, C42 and C44; **Figure 1**).

Absolute concentrations of C36, C37, C40, C42 and C44 were 0.105 ± 0.030 µM, 0.031 ± 0.011 µM, 0.017 ± 0.001 µM, 0.121 ± 0.007 µM and 0.023 ± 0.005 µM in EDTA plasma, respectively (mean of n = 6 ± standard derivation). Since accuracy and precision of recovery rates in EDTA plasma exhibit best results among three tested blood-derived specimens, CAAPs concentrations in lithium-heparin and serum were compared relative to EDTA plasma. Concentrations of CAAPs found in different matrices from same healthy donors revealed that the relative change of concentrations is consistent for serum compared to EDTA plasma (range of CV for five detected CAAPs: 2.5 - 8.7 %; **Figure 1****).** However, absolute mean serum concentrations differ between 94 - 131 % compared to EDTA, depending on the respective CAAP. C36, C37 and C42 measured in lithium-heparin plasma also showed acceptable variation in relative concentrations compared to EDTA plasma (11.1 %, 5.3% and 4 % CV, respectively) and comparable absolute concentrations compared to EDTA (between 105 and 106 %). However, C40 and C44 showed unacceptable high variations among individuals (43.4 and 23.1%) and mean concentrations were much lower in lithium-heparin plasma (82 and 55%) compared to EDTA.

### Example 5: Clinical applications

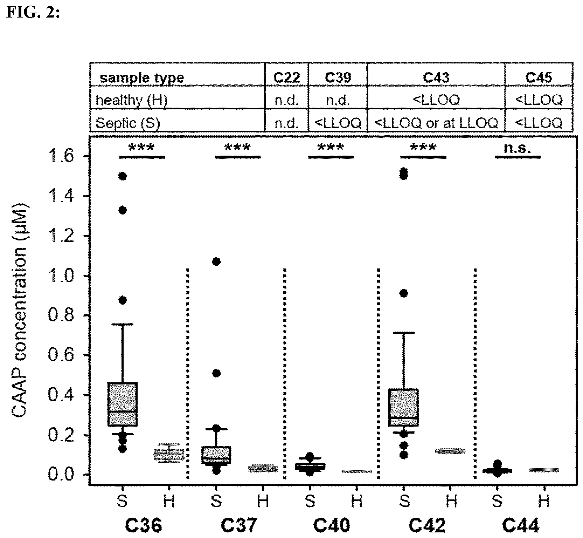

Since CAAPs can be endogenously generated from enzymatic activity that is associated with inflammation and by microbes frequently causing septic shock, a clinical patient's cohort suffering from sepsis was chosen to initially investigate CAAPs profiles during infectious-induced systemic inflammation. EDTA plasma was used because it showed best results among the tested blood-derived specimens during validation. Concentrations of C36, C37, C40 and C42 were significantly higher in the sepsis cohort compared to healthy control samples (P< 0.001, respectively, **Figure 2**). C44 in septic patient samples remains on healthy concentration level (P = 0.229). C22, C39, C43 and C45 were absent or below LLOQ.

Based on median values for each CAAP concentration, sepsis patients exhibit between 2.3-3.0 times higher concentrations of C36, C37, C40 and C42 than healthy individuals. Among the significantly increased CAAPs in sepsis samples compared to healthy individuals (**Figure 2**), we have investigated the validity of C36, C37, C40 and C42 as potential biomarker for sepsis using ROC analysis (table 9). C36 and C42 resulted in AUROC value of above 97% and high sensitives (97%) and specificities (100%), respectively. Classifications of C37 and C40 were less sensitive and specific for discriminating sepsis from healthy patients.

**Table 9: Accuracy of selected CAAPs for their potential to discriminate sepsis patients from healthy individuals. The data set used was the same as in Figure 2.**

| **CAAP** | **AUC** [95% CI] | **Cutoff^{#}** (µM) | **Sensitivity** (n⁺/n) | **Specificity** (n⁻/n) |
|---|---|---|---|---|
| C36 | 99% [98-101%] | 0.162 | 97% (35/36) | 100% (6/6) |
| C37 | 97% [92-102%] | 0.048 | 97% (34/36) | 71% (5/6) |
| C40 | 96% [91-102%] | 0.020 | 88% (35/36) | 50% (1/6) |
| C42 | 97% [92-103%] | 0.137 | 97% (35/36) | 100% (6/6) |

| | | | | |
|---|---|---|---|---|
| # Cutoffs are derived from Kolmogorov-Smirnov statistics. AUC: area under the receiver operating characteristics curve; CI: confidence interval; n⁺: measurements with positive test results; n⁻: measurements with negative test results; n: all measured values | | | | |

For the two most abundant CAAPs C36 and C42, we have also analyzed their plasma concentrations in septic patients during the first week of treatment at the ICU (**Fehler! Verweisquelle konnte nicht gefunden werden.,** n=27).

Mean C42 concentrations did not change over time whereas the majority of detected C36 tended to decrease. We have further analyzed the development of C36 and C42 concentrations towards striking features among the sepsis cohort and we found that three out of 27 patients exhibit opposite trends for C36 and C42 concentrations over time (patient ID 21, 31 and 49; **Fehler! Verweisquelle konnte nicht gefunden werden.**). For these three cases, C42 increases while C36 decreases over time.

### Example 6: Bioanalytical method validation

**Working range of calibrants** was defined based on CAAPs detected in samples from healthy donors (set to approximate lower limit of quantification) and in samples from septic patients (set to middle to high calibration range) which were re-analyzed from a previously work. Nine calibrants were used in total ranging from 0.01 µM to 1.5 µM thereby covering more than two orders of magnitude in concentration.

**Linearity** was assessed determining mean linear regression coefficients of each calibration curve from 0.01 to 1.5 µM in eight independent analytical runs. However, respective LLOQs (lower limit of quantification) and ULOQs (upper limit of quantification) were defined individually based on natural occurrence (see above) and results from validation parameter directly affected individual working ranges (carry-over, specificity). Four QC samples were prepared and measured in total (QC1, QC2, QC3, and QC4) to cover the entire range of calibration. However, three QCs for each of the respective analytes were used for method validation.

**Carry-over** was determined measuring pure albumin solutions prepared without internal standards (but equal amount and concentration of water/formic acid solution as regular internal standard solution, "blank albumin sample") directly after the respective highest calibrants. Peak areas were compared to peak areas from respective LLOQ samples (prepared with regular internal standard solution).

**Specificity** was assessed using single compounds at respective highest calibrant level which were prepared and calculated as described for carry-over. The specificity of analytes towards internal standards was determined using pure albumin solution with internal standard ("zero albumin sample").

**Accuracy and precision** was determined by back-calculated concentrations from repeated measurements of the same QC samples during one analytical run (*within-run,* n=5) and from freshly prepared samples during five independent analytical runs (*between-run,* n=5).

Due to the unavailability of analyte-free biological matrix, **selectivity** of analytes were assessed in pure albumin solution containing internal standards.

**Matrix effects** were determined calculating recovery rates and precision of each compound in human blood samples. Direct comparison between different anticoagulants (EDTA, lithium-heparin) and serum was performed using whole blood from the same human donor. Blood donations from volunteers were approved by the Ethics Committee of University Hospital Jena (Ethics number: 4619-11/15). Whole blood was collected in EDTA K₃, Lithium-Heparin Plasma-Gel and Serum Gel Z S-Monovettes (Sarstedt, Nümbrecht, Germany) from six healthy donors (3f/ 3m; age from 27 to 45 years), respectively, and prepared using standard centrifugal conditions (10 min., 2762 g, room temperature; model 5804R, Eppendorf AG) before storage at -80°C. To minimize pre-analytical interferences, HIL indices (hemolysis, icterus, lipemia) were measured in lithium-heparin plasma as well as serum (Architect ci8200, Abbott Laboratories, Abbott Park, IL, USA) and results from both measures were valued as within normal ranges (data not shown). All volunteers gave their written informed consent.

To determine **recovery rates in different blood matrices** spiked plasma was obtained by adding 5 µL of the spiking solution (peptide-mix standard in albumin: high [7µM] and low [1µM]) to 30 µL of different biological matrices from six healthy donors (EDTA plasma, lithium-heparin plasma and serum), respectively. Control samples ("0") were pure albumin solutions added to biological matrices. Measurements were performed in duplicates. To determine recovery rates, accuracy was calculated by peak area ratios of analyte to internal standard using following equation: (area ratio of spiked sample) - (area ratio of endogenous concentration))/ (area ratio of spiked albumin sample). To evaluate the consistence of matrix effect among individual donors, precision of peak area ratios of analyte to internal standard was investigated for six individual donors in each of the biological matrix.

To verify the **linear response** of CAAPs in different matrices, pool matrices consisting of equal volumes from each of the six donors was prepared and spiked with standard peptide mix (14µM stock) before serial dilutions in the same pool matrix were conducted.

Investigations of analyte **stabilities** were carried out using QC samples. Autosampler stability was determined by injecting each QC at the beginning of the run and 20 hours later during six independent runs. The CV (%) of the back-calculated concentration (n=2) was used to ensure autosampler stability. To determine stability of analytes after two cycles of freezing and thawing ("freeze/ thaw") as well as after chilling for 60 min. at room temperature ("benchtop") three QCs at two concentrations levels were used (QC2 and QC3), respectively. Samples were analyzed against freshly prepared QCs.

### Example 7: Study cohort

To initially determine CAAP profiles in sepsis patients, a well-characterized set of study samples provided by the Department of Anesthesiology and Intensive Care Therapy (Jena University Hospital) was chosen. The study protocol, Ethics Committee approval and collection of blood samples are described in a corresponding previous publication. All Patients were treated at the Intensive Care Unit (ICU) and blood samples were taken within 24 hours after septic symptoms emerged and within the following seven days. For data analysis and interpretation of CAAPs profiles, two subgroups were selected. Subgroup A includes all patients of whom blood samples were available within three days after sepsis onset. Subgroup B includes patients of whom blood samples were available at least three different days after sepsis onset and during their stay at the ICU. Patient characteristics are listed in table 10.

**Table 10: Patient characteristics. Severe sepsis/ septic shock was diagnosed according to the ACCP/SCCM criteria and corresponds to the Sepsis-3 definition.**

| | **Cohort, *n* = 48** |
|---|---|
| Age, median (range) | 65 (31-84) |
| Female, *n* (%) | 17 (35) |
| 28-day-survival, *n* (%) | 37(77) |
| SOFA at day of study admission^{#}, median (25-75 percentile range) | 10 (7-13) |
| Site of infection, *n* (%) | |
| Abdominal | 19 (39.6) |
| Pneumonia | 16 (33.3) |
| Soft tissue | 2 (4.2) |
| Primary bacteremia | 5 (10.4) |
| Endocarditis | 4 (8.3) |
| Urogenital | 2 (4.2) |
| **Microbial-positive infection,** *n* (%) | **29** (60) |
| Positive blood culture | 10 (34) |
| Multiple microbial-positive | 6 (21) |
| *S. aureus* | 5 (17) |
| *E. coli* | 6 (21) |
| *P. aeruginosa* | 3 (10) |
| *Enterococci* | 15 (52) |
| others | 7 (24) |
| **Subgroups** | **Cohort, *n* = 48** |
| Subgroup A, *n* (%) | 36 (75) |
| Subgroup B, *n* (%) | 27 (56) |

| | |
|---|---|
| ^{#} When SOFA was not available at the day of study admission, SOFA was taken from the next day (n=4). | |

### Example 8: Data processing and statistical analysis

Analyst Software (version 1.6.2 and 1.7.1) was used for mass spectrometer data acquisition and processing. The raw data were imported into the quantification wizard tool, peak integration was reviewed individually and results were generated in relation to the respective internal standard. A 1/x^{∗}x weighted quadratic regression was used to calculate the concentrations of each of the eighteen compounds.

To calculate final concentrations for each of the nine CAAP lengths, concentrations of C42^{WT} and C42^{SNP} were used as reference compounds for determining individual phenotypes of rs1303 polymorphisms (homozygote WT, homozygote SNP or heterozygote). A tolerance of maximum 30% coefficient of variance was accepted between concentrations of C42^{WT} and C42^{SNP} for the heterozygote type.

Graphs and statistical analysis were performed using SigmaPlot 14.0. Measurements are presented as boxplots (median, 10/90^{th} percentile) with error bars. For the classification of CAAPs as biomarkers, receiver operating characteristics analyses and cross tables were performed using SPSS statistics version 27 (IBM, New Armonk, NY).

### Example 9: Discussion

Since the role of CAAPs as biomarker has been proposed for many diseases, the method of the invention is of important and broad clinical relevance. Biomarker that allow for identifying and quantifying systemic inflammation are an essential tool not only for diagnosis but also for treatment of systemic inflammatory disorders but are rarely available yet.

Due to the role of C42 as biomarker in systemic infection and thus its potential application as sepsis biomarker in routine diagnostics, the method of the invention is focused on a rapid workflow. In contrast to the prior art, the present invention possesses a rapid workflow and optimized chromatographic conditions for eighteen CAAPs analytes. The entire process from sample preparation to quantitative results can be completed in less than one hour (30 min. sample preparation, 12 min. LC run). Working ranges were optimized based on results for carryover and specificity as well as concentrations detected in healthy and septic individuals. Concentration-dependent responses of all investigated CAAPs were linear across more than two orders of magnitude. Therefore, time-consuming steps for sample preparation (*e.g.* extraction or dilution), which are usually applied, are not necessary. In addition to the more efficient workflow, quantification of CAAPs in healthy individuals can now also be achieved (**Figure 1**) due to increased sensitivity of the present invention compared to the prior art. Finally, tests determining accuracy and precision (within-run and between-runs) of back-calculated CAAP concentrations using QC samples were successfully passed (table 6).

The detection of C36, C37, C40, C42 and C44 (**Figure 2**) are in agreement with knowledge from the literature as they have been reported to be generated human endogenously. The present invention is the first disclosure of a method for quantitatively measuring C-terminal cleavage products of alpha-1-antitrypsin in blood-derived samples.

In addition to the higher concentrations of C42 in septic patients compared to healthy individuals (**Figure 2**) our results demonstrate that C36, C37 and C40 seem to play a crucial role in systemic inflammation as well and thus might also have the potential to serve as sepsis biomarkers in blood-derived samples. And indeed, the validity of C36 and C42 as sepsis biomarker was confirmed by high sensitivity and specificity (table 9). C36 experiences the greatest change among CAAPs measured in septic patients compared to concentrations measured in healthy individuals (n=36, **Figure 2****).**

For three patients (ID 21, 31 and 49) we detected clearly opposite trends for the development of C36 (decrease) and C42 (increase) concentrations over time. Decreases of C36 is followed by high initial C36 concentrations; moreover all three cases were affected by pneumonia (site of sepsis infection) with very high SOFA scores at study admission (patient 21: 11; patient 31: 15; patient 49: 15). It is well known that increased activity of neutrophil elastase is involved in the pathogenesis of various lung diseases such as pneumonia. C36 can be formed by neutrophil elastase and has already been found *in vivo* in lung-correlated tissues. Further investigation and studies are needed to uncover potential correlations of CAAPs with, for example, site of infection, organ failure or specific enzymatic activities.

## Claims

1. A method for quantitatively measuring a concentration of a peptide panel in a biological matrix by mass spectrometry detection, wherein multiplex multiple reaction monitoring (MRM) scanning is applied, wherein the method comprises the steps a)-d):
a) sample collection,
b) sample preparation,
c) detection by mass spectrometry,
d) statistical analyses and quantification.

2. The method of claim 1 wherein sample preparation comprises mixing sample with internal standard and a sample solvent and centrifugation.

3. The method of any of the preceding claims wherein detection by mass spectrometry comprises LC-MS/MS analysis comprising the steps a)-:
a) mixing sample with internal standard and a sample solvent,
b) chromatographic separation,
c) mass spectrometry.

4. The method of claim 3 wherein the sample solvent is selected from the group comprising cooled methanol.

5. The method of claim 3 wherein chromatographic separation involves application of a gradient of solvents, wherein the solvents for gradient chromatography are selected from the group comprising formic acid in water/acetonitrile and water/methanol.

6. The method of any of the preceding claims wherein the biological matrix is human tissues selected from the group comprising spleen and placenta and/or exhaled breath/exhaled breath condensate (EBC) and/or human liquids selected from the group comprising whole blood, blood and blood fractions, serum, plasma, lymphatic fluid, urine, tears, saliva, sputum, bile, bronchoalveolar lavage fluid, pleural effusions, cerebrospinal fluid (CSF), nipple aspiration fluid, umbilical cord blood and punctate samples taken from, e.g., ascites or pleura or an extract of any of the aforementioned samples.

7. The method of any of the preceding claims wherein the peptide panel comprises one or more proteolytic degradation product peptides of a protein.

8. The method of claim 7 wherein the peptides are associated with a condition selected from the group comprising infection, inflammation, systemic inflammation, septic shock, sepsis, severe sepsis, cancer, pulmonary fibrosis, kidney diseases and allergies.

9. The method of claim 7 wherein the peptides are sepsis biomarkers.

10. The method of claim 7 wherein the protein is major human SERPIN alpha-1-antitrypsin (A1AT).

11. The method of claim 7 wherein the protein is proteolytically degraded by one or more proteases selected from the group comprising matrix-metalloproteinases (MMPs), serine proteases, cysteine proteases, metalloproteases, cathepsins, semi-alkine proteinase

12. The method of any of the preceding claims wherein mean accuracies are within ±20% of lower limit of quantification or wherein mean precisions are within ±20% of lower limit of quantification or wherein carry-over is less than ±5% of peak areas of the peptide's lower limit of quantification.

13. A method for the diagnosis, prediction or risk stratification for mortality or disease outcome of a subject that has or is suspected to have sepsis, comprising the steps of (a) - (c):
a) detection and quantification of a peptide panel by LC-MS/MS according to the method of claims 1-12,
b) wherein the measured concentration of peptides is correlated with an increased risk of mortality or poor disease outcome or diagnosis and/or prognosis and, wherein
c) said increased risk of mortality or poor disease outcome or diagnosis and/or prognosis is given if the concentration of peptides is below a certain cutoff value and/or the concentration of peptides is above a certain cut-off value.

14. A method for differentially diagnosing the causal infection of sepsis or septic shock in a subject, wherein the causal infections are selected from the group of (i) viral infection, (ii) bacterial infection, (iii) fungal infection or (iv) other microbial infections, said method comprising the steps of:
a) determining the level of CAAPs in a blood sample taken from said subject, and
b) correlating the level of CAAPs to (i) viral infection, (ii) bacterial infection, (iii) fungal infection or (iv) other microbial infections,
c) wherein said correlation of the level of CAAPs to (i) viral infection, (ii) bacterial infection, (iii) fungal infection or (iv) other microbial infections is given if the level of CAAPs is above or below a certain cut-off value.

15. A method for differentially diagnosing a disease in a subject, wherein the diseases are selected from the group of (i) non-infectious systemic inflammation, (ii) infectious systemic inflammation, (iii) infection, (iv) initial sepsis, (vi) different endogenous sepsis phenotypes and (vi) septic shock, said method comprising the steps of:
a) determining the level of CAAPs in a blood sample taken from said subject, and
b) correlating the level of CAAPs to (i) non-infectious systemic inflammation, (ii) infectious systemic inflammation, (iii) infection, (iv) initial sepsis, (v) different endogenous sepsis phenotypes and (vi) septic shock,
c) wherein said correlation of the level of CAAPs to (i) non-infectious systemic inflammation, (ii) infectious systemic inflammation, (iii) infection, (iv) initial sepsis, (v) different endogenous sepsis phenotypes and (vi) septic shock is given if the level of CAAPs is above or below a certain cut-off value.

16. A kit for diagnosis of sepsis or predicting the prognosis in a patient with sepsis, comprising:
a) standard peptides of the peptide panel of claim 1,
b) internal standards and
c) reference data and concentrations of the analytes for healthy patients and under disease conditions,
d) standard data showing the correlation between the concentration of peptides contained in samples and diagnosis and/or prognosis and optionally
e) a manual.
